# EUROPEAN PATENT APPLICATION

(11) **EP 1 118 670 A1**
(43) Date of publication of application: **25.07.2001**
(21) Application number: 99403061.7
(22) Date of filing: 07.12.1999
(51) Int. Cl.: C12N 15/86, C12N 5/16, A61K 48/00

(54) **Canine adenovirus vectors for the transfer of genes in targeted cells**

(71) Applicant: Genethon III, 91002 Evry Cedex (FR)
(72) Inventor: Kremer, Eric, 75012 Paris (FR); Chillon, Rodriguez Miguel, 08195 Barcelone (ES); Soudais, Claire, 92260 Fontenay aux Roses (FR); Boutin, Sylvie, 94140 Alfortville (FR); Peltekian, Elise, 75020 Paris (FR); Garcia, Luis, 93200 Saint Denis (FR); Vincent, Nathalie, 91250 Saintry sur Seine (FR); Danos, Olivier, 77300 Fontainebleau (FR)
(74) Representative: Palix, Stéphane

(57) **Abstract**

The invention relates to a Canine Adenovirus (CAV) vector obtainable by a process comprising the following steps :
a) co-transforming E. coli cells having recBC sbcBC phenotype by a first plasmid and a pre-transfer plasmid in conditions enabling their recombination by homologous recombination, in order to generate a transfer plasmid devoid from a functional E1 coding region, comprising the desired recombinant vector genome, wherein the first plasmid comprises the Inverted Terminal Regions (ITR) and the Packaging Signal (ψ) sequences of a CAV genome, and the pre-transfer plasmid includes the sequence whose insertion in the vector genome is desired, flanked by sequences homologous to sequences of the first plasmid surrounding the region of the first plasmid where the modification is desired,
b) isolating a DNA fragment essentially comprising the recombinant vector genome by enzyme restriction,
c) transfecting DK28Cre cells (CNCM 1-2293) that are rendered able to transcomplement this recombinant vector genome,
d) recovering and purifying the recombinant adenoviral particles produced.
The obtained vector may be used for the transfer of any gene of interest in target cells, especially in neuronal cells.

SEQUENCE LISTING

<110> GENETHON III

<120> CANINE ADENOVIRUS VECTORS FOR THE TRANSFER OF GENES IN
      TARGETED CELLS.

<130> B4308_GENETHON

<140>
<141>

<160> 7

<170> PatentIn Ver. 2.1

<210> 1
<211> 450
<212> DNA
<213> Canine Adenovirus Vector

<400> 1
catcatcaat aatatacagg acaaagaggt gtggcttaaa tttgggtgtt gcaaggggcg 60
gggtcatggg acggtcaggt tcaggtcacg ccctggtcag ggtgttccca cgggaatgtc 120
cagtgacgtc aaaggcgtgg ttttacgaca gggcgagttc cgcggacttt tggccggcgc 180
cccgggtttt tgggcgttta ttgattttgc ggtttagcgg gtggtgcttt taccactgtt 240
tgcggaagat ttagttgttt atggagctgg ttttggtgcc agttcctcca cggctaatgt 300
caaagtttat gtcaatataa cagaaacact ctgttctctg tttacagcac cccacctagt 360
cgactaaaaa acctcccaca cctccccctg aacctgaaac ataaaatgaa tgcaattgtt 420
gttgttaact tgtttattgc agcttataat                                  450

<210> 2
<211> 389
<212> DNA
<213> Canine Adenovirus Vector

<400> 2
catcatcaat aatatacagg acaaagaggt gtggcttaaa tttgggtgtt gcaaggggcg 60
gggtcatggg acggtcaggt tcaggtcacg ccctggtcag ggtgttccca cgggaatgtc 120
cagtgacgtc aaaggcgtgg ttttacgaca gggcgagttc cgcggacttt tggccggata 180
acttcgtata gcatacatta tacgaagtta tccggcgcgc cgggtttttg ggcgtttatt 240
gatttatgta tacatgggtg gtgcttttac cactgtttgc ggaatatgga gctggttttg 300
gtgccagttc ctccacggct aatgtcaaag tttatgtcaa tataacagaa acactctgtt 360
ctctgtttac agcaccccac ctagtcgac                                   389

<210> 3

<211> 396
<212> DNA
<213> Canine Adenovirus Vector

<400> 3
catcatcaat aatatacagg acaaagaggt gtggcttaaa tttgggtgtt gcaaggggcg 60
gggtcatggg acggtcaggt tcaggtcacg ccctggtcag ggtgttccca cgggaatgtc 120
cagtgacgtc aaaggcgtgg ttttacgaca gggcgagttc cgcggacttt tggccggata 180
acttcgtata gcatacatta tacgaagtta tccggcgcgc cgggtttttg ggcgtttatt 240
gattttgcgg tttagcgggt ggtgctttta ccactggaag atttagttgt ttatggagct 300
ggttttggtg ccagttcctc cacggctaat gtcaaagttt atgtcaatat aacagaaaca 360
ctctgttctc tgtttacagc accccaccta gtcgac 396

<210> 4
<211> 390
<212> DNA
<213> Canine Adenovirus Vector

<400> 4
catcatcaat aatatacagg acaaagaggt gtggcttaaa tttgggtgtt gcaaggggcg 60
gggtcatggg acggtcaggt tcaggtcacg ccctggtcag ggtgttccca cgggaatgtc 120
cagtgacgtc aaaggcgtgg ttttacgaca gggcgagttc cgcggacttt tggccggata 180
acttcgtata gcatacatta tacgaagtta tccggcgcgc cgggtttttg ggcgtttatt 240
gattttgcgg tttagcgggt ggtgctttta ccactgtttg cggaatatgg agctggtttt 300
ggtgccagtt cctccacggc taatgtcaaa gtttatgtca atataacaga aacactctgt 360
tctctgttta cagcacccca cctagtcgac 390

<210> 5
<211> 450
<212> DNA
<213> Canine Adenovirus Vector

<400> 5
catcatcaat aatatacagg acaaagaggt gtggcttaaa tttgggtgtt gcaaggggcg 60
gggtcatggg acggtcaggt tcaggtcacg ccctggtcag ggtgttccca cgggaatgtc 120
cagtgacgtc aaaggcgtgg ttttacgaca gggcgagttc cgcggacttt tggccggata 180
acttcgtata gcatacatta tacgaagtta tccggcgccc cgggtttttg ggcgtttatt 240
gattttgcgg tttagcgggt ggtgctttta ccactgtttg cggaagattt agttgtttat 300
ggagctggtt ttggtgccag ttcctccacg gctaatgtca aagtttatgt caatataaca 360
gaaacactct gttctctgtt tacagcaccc cacctagtcg actaaaaaac ctcccacacc 420
tccccctgaa cctgaaacat aaaatgaatg 450

<210> 6
<211> 401
<212> DNA

<213> Canine Adenovirus Vector

<400> 6
catcatcaat aatatacagg acaaagaggt gtggcttaaa tttgggtgtt gcaaggggcg 60
gggtcatggg acggtcaggt tcaggtcacg ccctggtcag ggtgttccca cgggaatgtc 120
cagtgacgtc aaaggcgtgg ttttacgaca gggcgagttc cgcggacttt tggccggata 180
acttcgtata gcatacatta tacgaagtta tccggcgcgc cgggtttttg ggcgtttatt 240
gatttatgta tacatgggtg gtgcttttac cactgtttgc ggaagattta gttgtttatg 300
gagctggttt tggtgccagt tcctccacgg ctaatgtcaa agtttatgtc aatataacag 360
aaacactctg ttctctgttt acagcacccc acctagtcga c 401

<210> 7
<211> 342
<212> DNA
<213> Canine Adenovirus Vector

<400> 7
catcatcaat aatatacagg acaaagaggt gtggcttaaa tttgggtgtt gcaaggggcg 60
gggtcatggg acggtcaggt tcaggtcacg ccctggtcag ggtgttccca cgggaatgtc 120
cagtgacgtc aaaggcgtgg ttttacgaca gggcgagttc cgcggacttt tggccggata 180
acttcgtata gcatacatta tacgaagtta tccggcgccc cgggtttttg ggcgtttatt 240
gattttgcgg tttagcgggt ggtgctttta ccactgtttg cggaagattt agttgtttat 300
ggagctggtt ttggtgccag ttcctccacg gctaatgtcg ac 342

## Description

The invention relates to the preparation and use of Canine Adenovirus (CAV) vectors, for the transfer of genes of interest in cells.

One purpose of the present invention is to provide means which can be used in gene therapy and especially which are adapted for the specific transfer of nucleotide sequences, including genes in determined targeted cells, in order for example to add a function to the cells or to correct the deficiency in the expression of genes involved in pathological states.

Gene therapy finds applications in diseases as diverse as heriditary disorders due to the alteration of a single gene, pathologies affecting the central nervous system, including degenerative neurological diseases, diseases resulting from enzymatic or hormonal deficiencies, auto-immune diseases, intracerebral or intraspinal tumors of any origin, peripheral tumors of nervous origin, treatment of pain, or in other diseases comprising inherited hematological diseases, overexpression or underexpression of metabolic enzymes, and cancers.

Viral vectors have been disclosed in the prior art in order to define means for the transfer of genes in cells. Among these vectors, retrovirus vectors and adenoviral vectors have been proposed though each has several drawbacks either as a result of their particular design or as a result of the biological environment surrounding their use in patients. Especially, but non exclusively, lack of efficiency of the infection of targeted cells by the vector particles, poor level of transduction of the target cells, lack of specificity for a determined cell and population in some cases, lack of security for the patient treated with these vectors have been observed.

Some difficulties have also been reported in designing the vectors, resulting from the deficiencies of the packaging cell lines used to produce viral particles for example as a result of an insufficient level of transfection or from contamination of the vector particles with Replication Competent Viruses (RCV).

For example, human adenoviruses type 2 and 5 were chosen as potential gene transfer vectors because of the significant amount of research performed on these serotypes. However, vectors derived from viruses that naturally infect and replicate in humans may not be the optimal candidates for therapeutic applications. Adenoviruses are ubiquitous in all populations and can be lethal in infants and immuno-compromised patients (5, 18, 24). Greater than 90% of the adult population has detectable levels of circulating antibodies directed against antigens from human serotypes (9, 32, 33). Phase I trials using human adenovirus vectors have yielded conflicting results (8, 21, 41). A difference in humoral immunity that is directed against the vector capsid might explain, in addition to other factors, the variability between and within these studies. Furthermore, when repeat administrations were attempted (7, 42) transgene activity was not detected. Studies aimed at immuno-tolerisation of mice, for the primary or repeat delivery of human adenoriral vectors, are interesting from the immunological standpoint, but may have limited practical use in the clinic. Will immuno-tolerisation of patients to adenoriral vectors activate latent, more virulent, serotypes? Concomitantly, there are other drawbacks associated with human-derived adenoriral vectors. Greater than 95% of a healthy cohort had a long-lived CD4⁺ T-cell response directed against multiple human adenovirus serotypes (14). These data imply that adenovirus serotypes switching (27) may have limited advantages. Furthermore, replication-competent adenoviruses (26) can potentially contaminate human adenovirus-derived vector stocks, including gutless adenoriral vectors (16, 22), while E1-region positive vectors are a potential contaminant in E1/E4 deleted vectors (40). In addition, recombination of the vector with a wild type adenovirus, producing a replication-competent adenovirus harbouring a transgene, still remains a theoretical risk with early generation vectors.

In order to address these issues, nonhuman adenoviral vectors have been generated, starting from the Manhattan strain of canine adenovirus type 2 (20). However, according to the reported experimental work, it was impossible to generate a recombinant CAV vector derived from this serotype, that was not significantly (>99%) contaminated with RCV. Replication-competent nonhuman adenovirus vectors from bovine, ovine and fowl have been described (28, 29, 37, 39) and currently appear useful as vaccines in nonhumans. In order to generate vectors for gene transfer in the clinic, the potentially oncogenic CAV-2 E1 region must be deleted from the vector stock, and a CAV-2 E1-transcomplementing cell line must be generated in order to propagate the vectors.

CAV vectors and derivatives are especially useful in the absence of pre-existing humoral immunity that can neutralise transduction. The inventors have shown that sera from a majority of a random healthy cohort contain significant amounts of neutralising adenovirus 5 antibodies, but not neutralising CAV-2 antibodies.

The inventors have shown in the present invention, that preparation of improved CAV vectors may be achieved, having recourse to different means, for instance in choosing a type of CAV strain, or/and selecting particular cell lines for transcomplementation of the vector genome in order to produce stocks of vectors and/or including the definition of the process steps to be carried out.

The inventors have accordingly defined new vectors that can be used in gene transfer.

The vectors which were generated in accordance with the invention, have been shown to present improved properties with respect to the vector disclosed in (20). In a particular embodiment, they are especially improved regarding contamination by RCV.

Moreover, the results obtained by the inventors following the transduction of various cells of different origins, *in vitro* or *in vivo,* have shown that adenovirus vectors can be designed starting from CAV, especially from CAV-2, that enable transfer of genes in targeted tissues or cells.

Thus, the invention relates to vectors comprising sequences derived from CAV genomic sequences, cell lines and especially cell lines of canine origin, for the production of said CAV (also designated vector particles) and further relates to the use of these vectors for the transfer of nucleic acid sequences in cells. Said transfer may be stable or temporary.

According to a first definition of the invention, a CAV vector is obtainable by a process comprising the following steps :
a) co-transforming E. coli cells having recBC sbcBC phenotype by a first plasmid and a pre-transfer plasmid in conditions enabling their recombination by homologous recombination, in order to generate a transfer plasmid devoid of a functional E1 coding region, comprising the desired recombinant vector genome, wherein the first plasmid comprises the Inverted Terminal Regions (ITR) and the Packaging Signal (ψ) sequences of a CAV genome, and the pre-transfer plasmid includes the sequence whose insertion in the vector genome is desired, flanked by sequences homologous to sequences surrounding the region of the first plasmid where the modification is desired,
b) isolating a DNA fragment essentially comprising the recombinant vector genome by enzyme restriction,
c) transfecting DK28Cre (CNCM 1-2293) cells that are rendered able to transcomplement this recombinant vector genome,
d) recovering and purifying the recombinant adenoviral particles produced.

The transfer plasmid resulting from the homologous recombination in E. coli cells is devoid of a functional E1 coding region, therefore requiring transcomplementation in a cell line. The term "functional' refers to the viral function of the E1 region.

The expression "modification" includes the replacement of the region of the first plasmid as a result of the recombination, and includes further the substitution of part of said region, in order to clone (insert) the sequence of interest (heterologous sequence) in the final transfer plasmid.

The term "homologous" relates to sequences which are identical in their nucleotide sequence, or to sequences which comprise differences in the nucleotides but can however be recombined when they are present on the first and pre-transfer plasmids.

The above referenced DK28Cre cell line will be described in details in the following pages. For illustration purposes, step (d) of the above defined process is described in the examples and especially can be achieved in treating the cleared lysate on a step CsCI gradient and centrifugating to isolate a band corresponding to the recombinant adenoviral particles and further purifying on a CsCI isopycnic gradient.

In a preferred embodiment of the invention, the CAV genomic sequences are derived from CAV-2 strain Toronto A26/61.

In another aspect, the invention relates to a CAV, which comprises:
a) a nucleotide sequence derived from a canine adenovirus-2 strain Toronto A26/61 genomic sequence, comprising the left and right ITR and Ψ sequence, said nucleotide sequence being devoid of the E1 coding region of the CAV genome, and
b) an expression cassette comprising a heterologous nucleotide sequence, said nucleotide sequence being under the control of regulatory sequences including a promoter sequence.

According to the above-defined preferred embodiment of the present invention, the CAV genome is prepared starting from the CAV-2 strain Toronto A26/61. This CAV strain is available at the ATCC under n° VR-800 or and a sequence of Toronto strain is available in Genbank under accession number 477082.

When the CAV vector of the invention comprises essentially all the nucleotide sequences encoding the viral functions of the CAV strain, especially those of the CAV-2 Toronto A26/61 strain, it remains however devoid of the E1 region.

The above defined vector is used for the transfer of the heterologous sequence contained in the expression cassette in target cells.

The genome vector used to prepare the vector is preferably cloned in a plasmid or alternatively in cosmids, YAC, or other DNA constructs.

The present invention therefore relates to novel CAV vectors, and to the genomes of these vectors.

According to the above definition, the nucleotide sequence, which is designated as the "heterologous sequence", is a sequence which is not naturally contained in the CAV genome and whose transfer is desired in target cells, either *in vitro* or *in vivo.*

The heterologous sequence is placed under the control of regulation sequences including a promoter sequence, which are not those of the specific CAV genomic sequence used for the preparation of the vector. The defined expression cassette can be inserted in any region of the CAV genomic sequence which is contained in the vector, provided this insertion does not affect the function of the proteins encoded by the CAV genomic sequence.

As far as the expression cassette is concerned, the invention is directed to a cassette wherein the expression of the heterologous nucleotide sequence is driven by a viral promoter, for instance, the SV 40 early promoter or CMV promoter. The promoter can be also a non viral promoter, or can be a promoter of cellular origin, for example the EF1-α promoter. It may be a constitutive or an inducible promoter, it may be a tissue-specific promoter.

If the vector genome of the invention is prepared in such a way that the only deleted sequence of the CAV genome is the E1 region, the expression cassette will be advantageously prepared in order to finally obtain a vector which has substantially the same size, for instance between 70 to 110% of the size of the CAV genome used, advantageously of the Toronto strain.

The nucleotide sequence (heterologous sequence) contained in the expression cassette can be any sequence of interest including any sequence of therapeutic interest, whose transfer in targeted cells including in cells of a patient, would be desired. If appropriate, several heterologous sequences can be inserted in the cassette or/and several cassettes can be inserted in the vector. A "heterologous sequence" according to the invention can be a coding sequence or a non coding sequence, including all regulatory sequences at the post-transcriptional, translational or transport levels.

Within the definition of this nucleotide sequence of the expression cassette one can mention any sequence that would be useful to provide targeted cells with a new function or sequences which are to the contrary capable of affecting and especially deleting a function in targeted cells. It could also consist of antisense sequences which would be used in order to modify the function of determined genes in targeted cells or sequences that could be recombined with genes of the targeted cells.

This nucleotide sequence contained in the expression cassette can be of experimental or therapeutic interest. More particularly, this nucleotide sequence can be aimed at gene transfer into cells of neuronal type, neural progenitors or differenciated neurons of any origin, *in vitro* and *in vivo.* As example, it can be the gene of tyrosine hydroxylase or glial derived neurotrophic factors, genes of neurotransmitter molecules, neuromodulators, neuropeptides, or their precursors (pre-pro-enkephaline for example), genes of enzymes implicated in neurotransmission: glutamic acid decarboxylase, (GAD), tyrosine hydroxylase (TH), choline acethyl transferase (ChAT).

Genes of cellular receptors or receptors sub-units can be used as well: neurotransmitter receptors (ionotropic or metabotropic glutamate receptors), receptors to neuromodulators (acethylcholine or dopamine or serotonine different receptors), receptors to neuropeptides (opioid receptors), growth factors, hormones, cytokines, neurotrophic factors (TrkA, TrkB, TrkC receptors for the neurotrophine family, respectively for Nerve growth Factor (NGF), Brain Derived Neurotrophic Factor (BDNF), Neurotrophic Factor 3 (NT3).

Other genes of interest are genes of enzymes implicated in metabolic pathways: Super Oxyde dismutase (SOD), genes of enzymes implicated in metabolic disorders (glucuronidase, adenosine deaminase for example), or genes of neurotrophine family molecules (NGF, BDNF, NT3) and genes of other neurotrophic factors (cyliary neurotrophic factor, glial cell derived neurotrophic factor), of cytokines (interleukines).

Genetic sequences leading to the synthesis of a fusion protein, for example with the aim to obtain the secretion and the delivery of the factor of interest to cells located in innervated structures or to tumoral cells of any origin located in the brain and the spinal cord, can be used as well.

The ITR and packaging sequences contained in the vector genome are necessary for the replication of the vector genome and for its packaging to produce vector particles after transfection of transcomplementing cells with a DNA molecule (for instance a restricted plasmid) comprising this genome.

The above definition of the CAV vector, appropriate for the transfer of a heterologous sequence in target cells, may encompass vectors whose genome contains large deletions in the nucleotide sequence originating form the CAV genome, including resulting in deletion, of all the regulatory and coding sequences of said CAV genome to the exception of the sequences necessary for the replication and for the packaging of the vector (so-called "gutless" vectors).

The invention thus relates in a particular embodiment, to a CAV vector wherein a substantial part of the nucleotide sequence originating from the CAV genome is deleted. In a particular embodiment the gutless vector genome comprises less than 3 % of the CAV genome, being the ITR and ψ.

To achieve an efficient packaging and stability, the gutless vector genome size is preferentially between 70% and 110 % of that of the wild type virus. Therefore, additional sequences, called "stuffer" sequences, must be inserted in gutless backbones.

The stuffer sequences can be any DNA, preferably of mammalian origin. In a prefered embodiment of the invention, stuffer sequences are non coding sequences of mammalian origin, for example intronic fragments. Advantageously, these fragments contain matrix attachment regions (MARs).

The stuffer sequence, used to keep the size of the gutless vector a predetermined size can be any mammalian sequence noncoding as well as one containing sequences that allow the vector genome to remain stable in dividing or nondividing cells. These sequences can be derived from other viral genomes (e.g. Epstein bar virus) or organism (e.g. yeast). For example, these sequences could be a functional part of centromeres and/or telomeres.

For instance in a particular embodiment, depending upon the size of the deleted sequences of the CAV genome, additional stuffer sequences can be inserted in the gutless vector in order to generate a vector having a size which is approximately the size of the CAV genome though a difference in size of the helper and gutless genomes of more than 6 kb can advantageously be maintained, in order to separate the two vectors by CsCI buoyant density.

In order to propagate largely deleted or gutless vectors, a helper vector is needed to transcomplement the viral functions of the CAV virus which have been deleted in the gutless vector genome. Optimized helper vectors have been designed by the inventors, whose encapsidation will be hindered when used in appropriate cells expressing the Cre recombinase.

According to a particular embodiment of the invention, the left and right ITR and ψ sequences are derived from the same CAV strain. According to another embodiment, these sequences are derived from different canine adenovirus strains.

A preferred vector according to the invention is one which replies to anyone of the above definitions especially, or to any combination of the above disclosed characteristics. In a particular embodiment, a vector of the invention is characterized in that the left ITR which it contains is comprised in the fragment extending from nucleotide 1 to nucleotide 411 of the genomic sequence of the CAV-2 Toronto strain, said fragment containing the left ITR and ψ sequences.

According to another embodiment of the invention, the CAV genome is replying to anyone of the above definitions or to any combination of the above definitions, and is characterized in that the left ITR which it contains is comprised in the fragment extending from nucleotide 1 to nucleotide 352 of the genomic sequence of the CAV-2 Toronto strain.

The use of a fragment containing the ITR and Ψ sequence of the genomic sequence of the Toronto strain, which is contained in the nucleotide sequence extending from nucleotide 1 to nucleotide 352 can be advantageous since it can prevent overlaps in the E1A region with the sequences of the CAV genome which are contained in the transcomplementing cell line. Such overlappings are especially advantageously prevented with the sequence used for transcomplementation of the E1 region, in order to avoid production of replication competent particles or E1-containing particles, by homologous recombination.

In another particular embodiment of the invention, the gutless vector genome comprises at least two ψ sequences derived from said nucleotide sequence of CAV genomic sequence included in the vector, in order to favour its packaging.

The inventors have especially generated CAV vectors derived from the Toronto A26/61 strain (also designated by Toronto strain), that may advantageously be produced in E1-transcomplementing cell lines derived from canine cells. The CAV vectors of the invention can be grown to high titres, for instance up to or even higher than 10¹³ particles/ml, they are replication-defective in canine cells. Advantageously, they are further replication-defective in human cells that have been shown to be able to transcomplement an E1-deleted human adenovirus vector. The property of the CAV vectors of the invention, to be replication-defective can be illustrated by the example that a CAV vector contains less than 1 replication - competent particle in 2.10¹¹ viral particles. An assay is described in the examples to illustrate how this property can be evaluated.

CAV vectors encompassed within the definition of the invention further especially those comprising the CAV-2 Toronto strain genomic sequence devoid of the E1 coding region, give encouraging results after having been tested in a) *in vitro* in order to determine their ability and efficacy to transduce human-derived cell lines compared to a human adenovirus vector; b) for the lack of replication-competent CAV-2 contaminating the stocks of particles produced, and c) for the particle to transduction unit ratio.

The invention also relates to CAV helper vectors which are useful for transcomplementation for the CAV vector genomes described here above, when the latter are devoid of the sequences encoding the necessary viral functions, especially when they are gutless vector genomes. The helper vector is used to provide viral functions which have been deleted from the gutless vector containing the expression cassette.

A particular CAV helper vector can be derived from the above disclosed CAV vectors, provided that lox sequences are inserted in the CAV genome in order to enable the deletion of the ψ sequence of said CAV genome when the vector is contacted with a Cre enzyme.

Alternatively, the Cre-lox system can be replaced by any functional equivalent thereof, allowing homologous recombination, for example FLP/FRT or other site-specific recombination systems.

The invention also relates to ψ sequence mutated in the helper vector, when such a vector is needed. Such modifications are illustrated in the following examples and are designed to hinder the packaging of the helper genome, in order to reduce its contamination in gutless vector stocks.

According to a particular embodiment of the invention, this helper vector can be devoid of any non-viral expression cassette.

The invention concerns therefore CAV vectors which consist of recombinant CAV particles that contain a CAV genome replying to one or several of the of the above definitions.

Especially, the invention relates to CAV vectors having a vector genome derived from the Toronto strain, in accordance with the above-given definitions of the CAV vector genome of the invention. The Toronto strain is a wild strain, which has not been attenuated, contrary to the Manhattan strain. This may be part of the reasons why the Toronto strain is more efficient than the Manhattan strain for the generation of CAV vector. Other wild type strains or substrains can be used within the scope of the invention.

CAV vector particles for the transfer of a nucleotide sequence in target cells according to the invention comprise an expression cassette comprising the nucleotide sequence to be transferred, wherein said cassette is cloned in a nucleotide sequence derived from the genomic sequence of a CAV strain, to constitute the vector genome.

A particular vector of the invention is the CAVGFP vector prepared with serotype 2 Toronto strain A26/61 which has been deposited at the CNCM on August 16, 1999 (Collection nationale de culture de microorganismes, Paris-France) under n° 1-2291. In this CAVGFP vector, the E1 region is deleted from bp 352-2898 and replaced by an expression cassette containing the CMV early promoter driving expression of enhanced green fluorescent protein (GFP) cDNA, followed by a polyA signal from SV40. The vector is replication-defective in all cell lines tested except DK/E1-28 and its derivatives.

This vector contains an expression cassette which comprises the gene expressing the GFP protein.

This expression cassette can be modified in order to substitue the gene encoding GFP by a nucleotide sequence of interest, for experimental purposes, or for therapeutic purposes.

Vector particles can be obtained especially by a process comprising the transfection of the CAV vector genome replying to the definitions which have been set forth above, in a transcomplementing cell line. This cell line is preferably of canine origin.

Especially, the invention concerns particular transcomplementing cell lines which have been shown to be efficient for the production of CAV vector particles of the invention, said cell lines being capable of expressing the E1 region of the CAV genome, especially the E1 region of the Manhattan strain or alternatively of the Toronto strain.

For example, such a cell line named DK/E1-28Z, is a Dog Kidney (DK cell line) stably expressing the E1 region of the genomic sequence of a CAV-2 Manhattan strain from nucleotide 352 to nucleotide 2898 (Genbank seq. JO 4368) (20) and stably expressing Neomycin and Zeocin resistance gene (20), it has been deposited at the CNCM on August 16, 1999, under n° I-2292.

This cell line can be modified especially by modifying the selection genes which it contains.

Another preferred transcomplementing cell line for the purpose of the invention is the DK cell line which further expresses the Cre recombinase. This cell line can be used with a helper vector containing lox sequences flanking its packaging signal.

This cell line has been deposited as DK28Cre at the CNCM under n° I-2293 on August 16, 1999. DK28Cre cell line is composed of DK cells stably expressing Neomycin and Zeocin resistance gene (20) plus E1 region from CAV-2 Manhattan strain from 322-2898 (Genbank sequence JO 4368) (20) and Cre recombinase (52).

The invention also concerns the use of the above-described means for the transfer of nucleotide sequence of interest especially for the transfer of genes in targeted cells. Said transfer can be made in a first step *in vitro* or alternatively can be directed *in vivo.*

Tests *in vivo* have been carried out, which show that CAV vectors of the invention can effectively transduce mouse airway epithelia when delivered intranasally. When injected in the brain, the CAV vector of the invention can have a strict neuronal tropism, said targeted transfer in determined cells being confirmed by injection in muscle which leads to a preferential transduction of motoneurons. The inventors have especially shown that the obtained vector of the invention is capable of specifically transferring gene in targeted cells such as neuronal cells.

The expression "treatment", applying for example to cells, comprises providing said cells with CAV vectors of the invention, in order to transfer the heterologous sequence contained in the CAV vector, to the cells, thereby modifying the cells and/or, their properties and/or functions.

Therefore, the CAV vector of the invention can be used for the preparation of a therapeutic composition for the treatment including modification of neuronal cells.

Particularly, the described properties of the CAV vectors opens the possibility to obtain local and neuron restricted trangenesis (including knock-in experiments) of central nervous system structures at any time of the development (injection in foetuses), or in the adult, providing a tool of value for any fundamental or therapeutic study.

With special concern for therapeutic strategies, whatever the level of investigation (experimental, preclinical or clinical), the means described herein allows some specific approaches due to its particular interaction with neurons. It particularly opens the possibility of using the neuro-anatomical connections either for the delivery of the therapeutic gene to neurons of a defined structure and/or for the delivery of a therapeutic factor synthesized by the transduced neurons and delivered at their neuritic and axonal endings.

Said treatment can especially be performed in using the therapeutic composition for the targeted administration of a nucleotide sequence of therapeutic interest in neuronal cells.

However, the use of this vector to transduce other cell types by other means of injection is not excluded.

The invention also concerns a process for the preparation of a CAV according to the invention, said process comprising the steps of:
a) co-transforming E. coli cells by a first plasmid and a pre-transfer plasmid in conditions enabling their recombination by homologous recombination, in order to generate a transfer plasmid devoid of a functional E1 coding region, comprising the desired recombinant vector genome, wherein the first plasmid comprises the ITR and ψ sequences of a CAV genome, and the pre-transfer plasmid includes the sequence whose insertion in the vector genome is desired, flanked by sequences homologous to sequences surrounding the region of the first plasmid where the modification is desired,
b) isolating a DNA fragment essentially comprising the recombinant vector genome by enzyme restriction,
c) transfecting cells that are able to transcomplement this recombinant vector genome, deleted in the E1 region,
d) recovering and purifying the recombinant adenoviral particles produced.

The E. coli cells used for the homologous recombination are selected for their recombination properties: they are preferentially recBC and sbcBC phenotype (47-49).

The above process for the generation of CAV vectors can be carried out in order to prepare any of the above defined CAV vector genomes. Therefore, the above first and pre-transfer plasmids will be designed in order to comprise the sequences of the CAV genome described in the above definitions and the expression cassette that shall be contained in the CAV vector genome as a result of recombination in E. coli.

The transcomplementing cell lines of the invention are appropriate to carry out the above process.

Said process can also be used, in a particular embodiment of the invention, for the production of largely deleted CAV vectors (gutless vectors) in substituting the above step c), by the following step:
- transfecting E1-transcomplementing cells with two plasmids, one with deletion in the E1 region and the second containing less than 3% of the CAV genome, said sequence being the ITR and ψ at each end of the gutless vector genome.

### Legend of the figures

Figure 1: Schematic representation of the pZeoCre plasmid

Figure 2: Schematic representation of the procedure used for the screening of Cre-expressing cells.

Figure 3: Generation of CAVGFP.
ptGFP was generated by homologous recombination in *Escherichia coli* BJ5183 using *Swa* I-linearised pTG5412 and a 4.7 kb *Bgl II/Fsp* I fragment from pCAVGFP (nucleotide position are from CAV-2, N.B. not drawn to scale). *Not* I digested ptGFP was transfected into DK/E1-1 cells and amplified 5-6 times on E1 transcomplementing cells and purified as described.

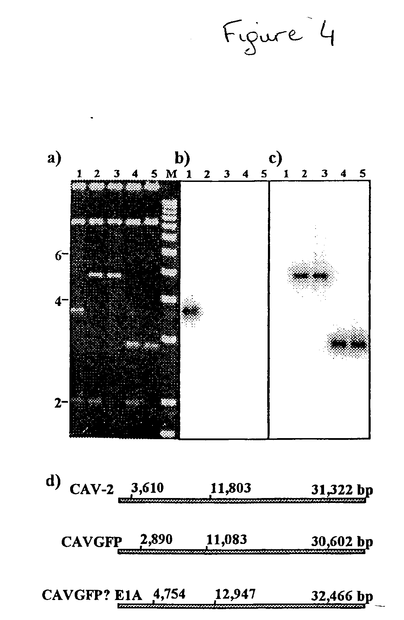

Figure 4: Digestion and Southern blot analysis of CAVGFP and CAVGFPΔE1A DNA.
a) Vector or plasmid DNA was digested with *Eco*R I and *Not I,* (in order to remove the 2 kb pPolyll backbone from the terminal fragments seen in lanes 1, 2 and 4), electrophoresed through a 0.7% agarose gel and stained with ethidium bromide:
a) 500 ng of (1) pTG5412, (2) ptGFPΔE1A, (3) CAVGFPΔE1A (4) ptGFP, and (5) CAVGFP. M denotes the 1 kb DNA ladder (GIBCO). Southern blot analysis, using b) a fragment of the E1A region or c) GFP cDNA as the radiolabelled probe, d) Location of the EcoR I sites and the fragment sizes in the vectors.

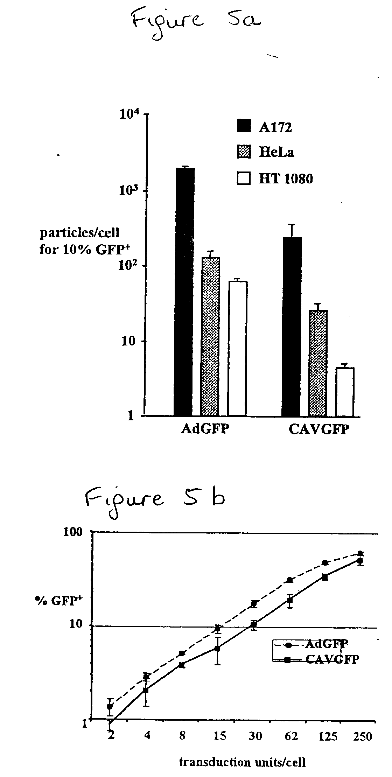

Figure 5: Quantitative analysis of transduction efficiency in human cells: CAVGFP vs. AdGFP.
a) A172, HeLa and HT 1080 cells were infected with each vector and assayed for GFP expression 48 hours post-transduction. The data represent the amount of vector required to generate 10% GFP positive cells/well expressed in input particles/well. Data are mean of 5 experiments ± SD. b) HeLa cells incubated with increasing number of particles of CAVGFP and AdGFP and analysed by FACS 24 hours post-transduction. The data are the mean ± the SD of triplicate samples.

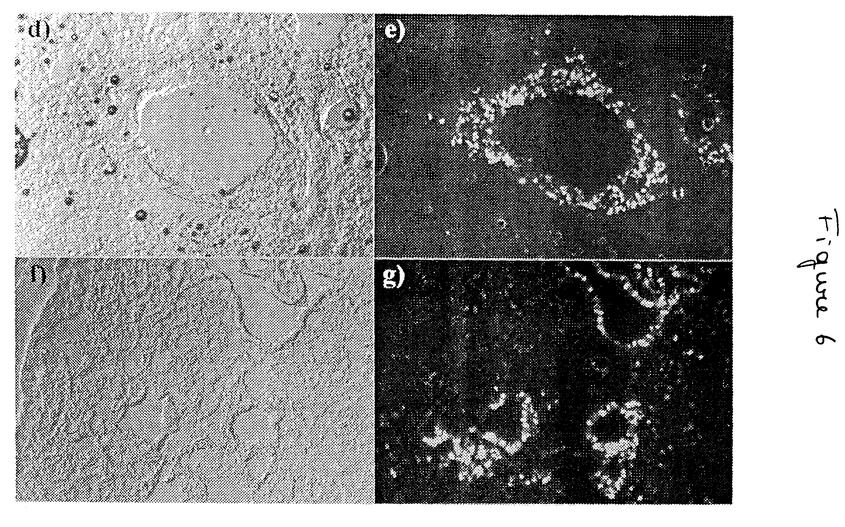

Figure 6: *In vivo* transduction of the airway epithelia in mice using CAV vectors.
10¹¹ particles, CAVGFP and AdGFP was delivered intranasally in BALB/c mice and assayed 3 or 4 days later. GFP expression in distal airways from CAVGFP and AdGFP d and f) phase contrast and e and g) GFP expression, respectively.

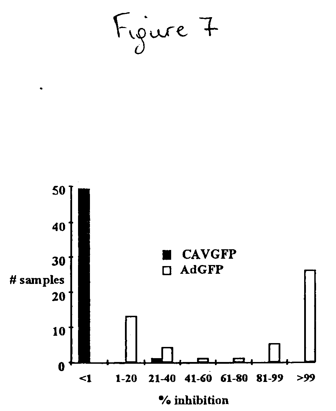

Figure 7: Pre-existing humoral immunity.
Sera from healthy blood bank donors (n=50) were assayed for the presence of neutralising CAV-2 antibodies. In this assay only one sample was partially able (~24%) to inhibit CAVGFP transduction while 26/50 samples completely inactivated AdGFP transduction.

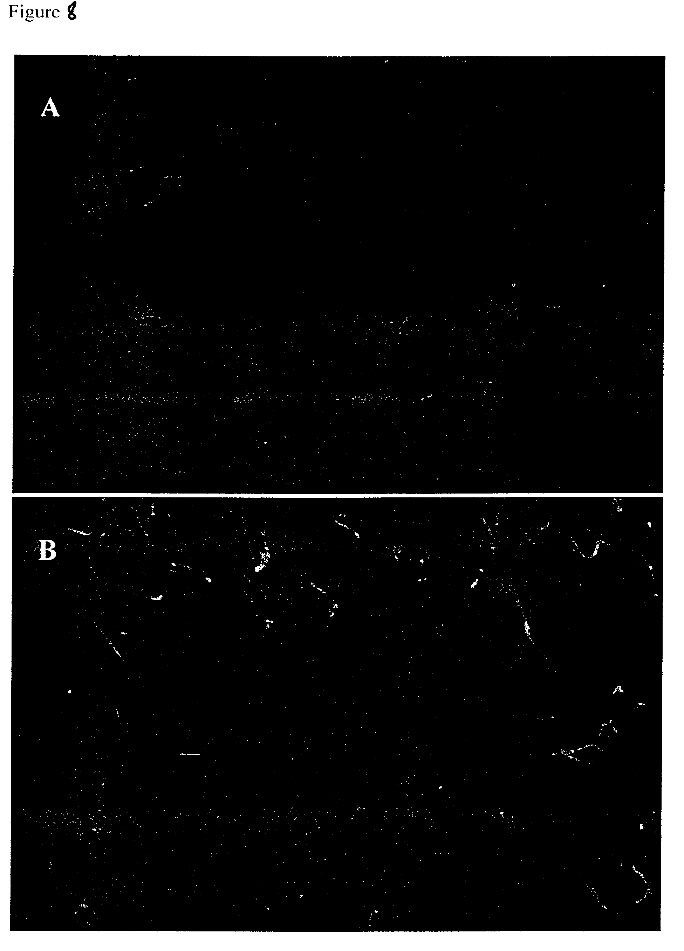

Figure 8: Coronal sections of rat hippocampus showing the site of injection
A : GFP positive neurones of the dentate gyrus.
B : Same secition as A : Immunohistochemistry for the astrocyte specific protein GFAP (glial fibrillary acidic protein). There is no detectable colocalisation of the two markers.

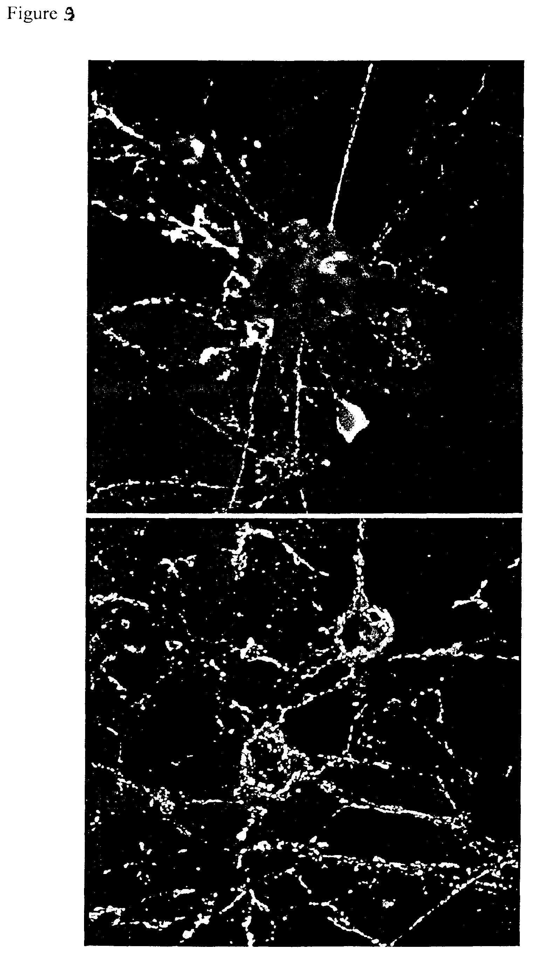

Figure 9: Rat foetal spinal cord explants (14 days) co-cultured with human muscle cells (CHQ5 cell line).
A : Binding of conjugated CAV particles (red) on neuritic and axonal processes.
B : Confocal microscopy image of neuronal interconnexions, showing that the binding is localised on axonal and neuritic processes and not found on the neuronal cell body.

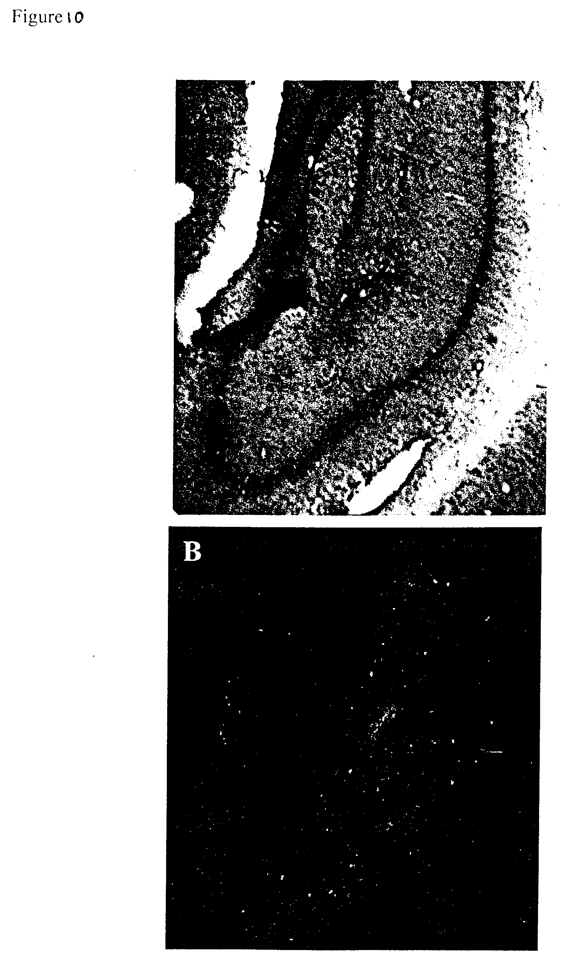

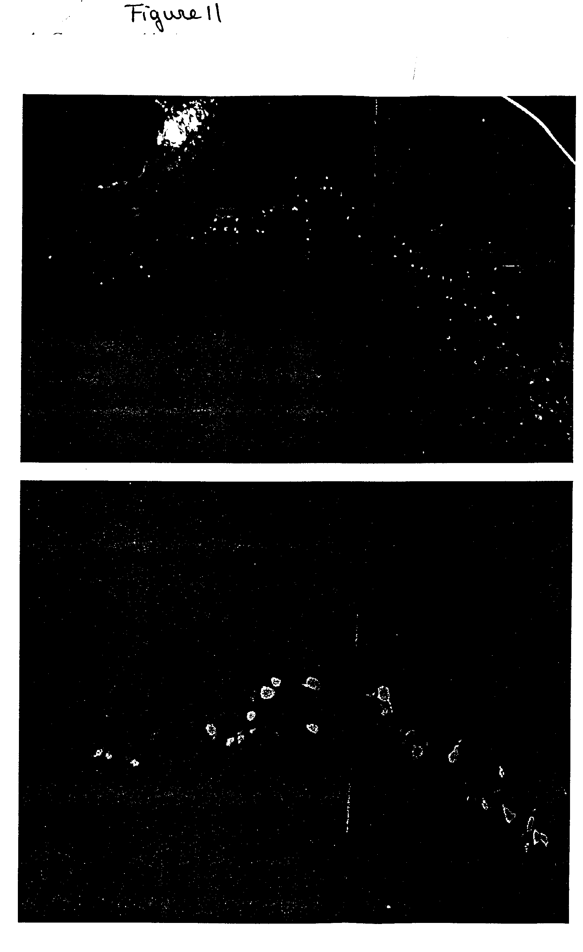

Figure 10: Coronal sections of rat dorsal hippocampus 15 days after injection of 10⁸ particles of CAV vector.
A : Cresyl violet coloration visualising the neuro-anatomical structure.
B : Location of the GFP staining throughout the Hammon's Horn and the dentate gyrus. Note the staining of the neuritic network.

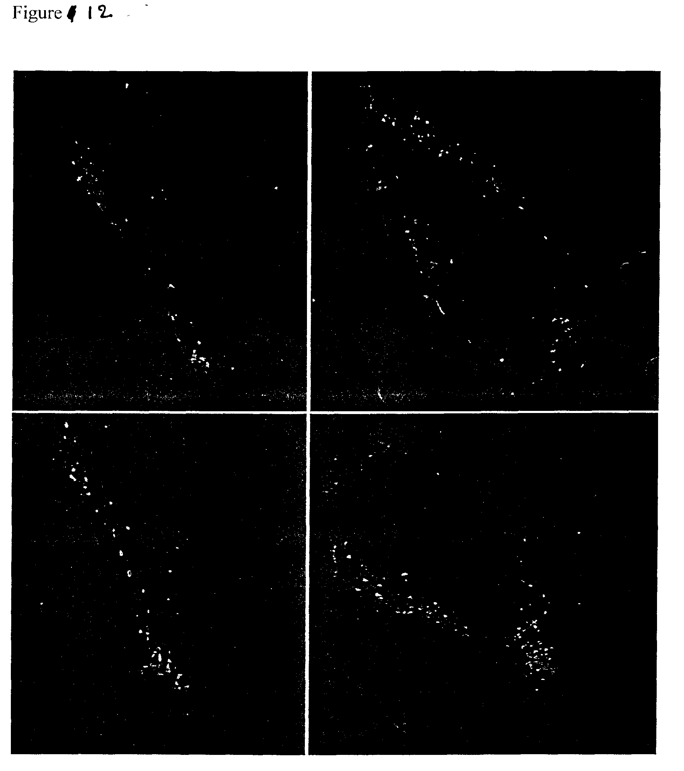

Figure 11: Enthorinal cortex; coronal sections.
A : 1h post-injection: Fluorescent conjugated particles of CAV vectors in juxtanuclear location in neurons of the entorhinal cortex. These particles were taken up from the nerve terminals and retrogradely transported after injection in the dentate gyrus.
B : 15 days post-injection; neurons of the entorhinal cortex, positive for the GFP, retrogradely transduced after injection in the dentate gyrus.

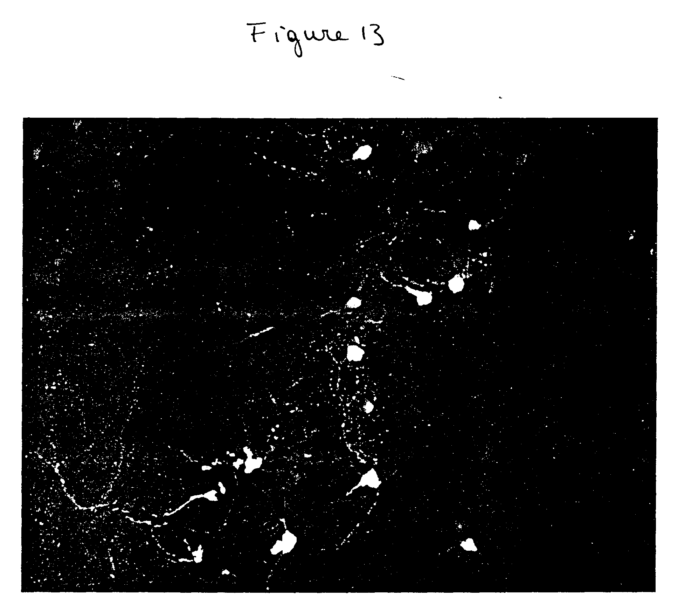

Figure 12: Substantia nigra compacta: Retrogradely transduced neurons after injection into the striatum. These four pictures are representative of a rostocaudal transduced area of 900µm.

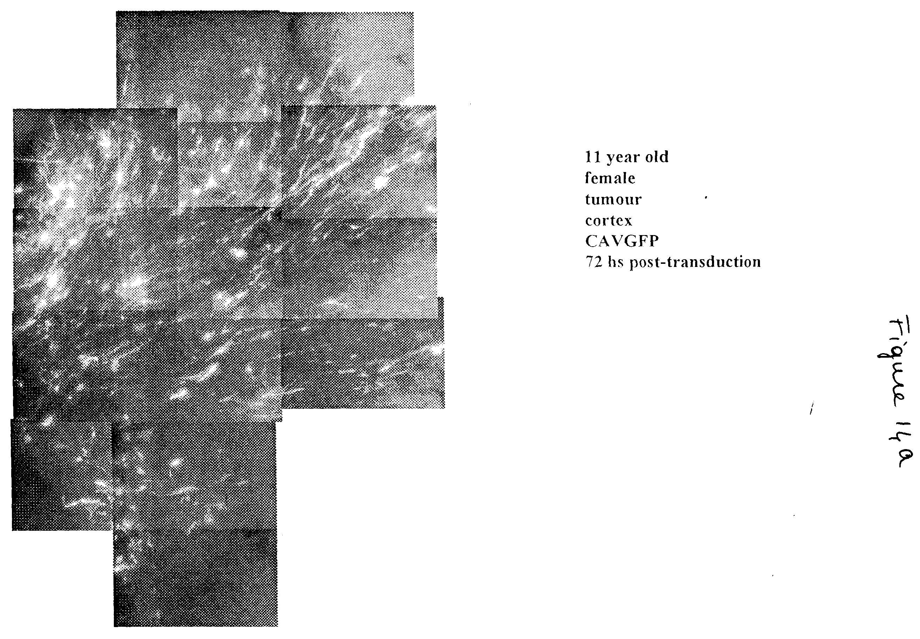

Figure 13: Somato-sensory cortex neurons, GFP positive, after injection of 10⁸ particles of CAVGFP.

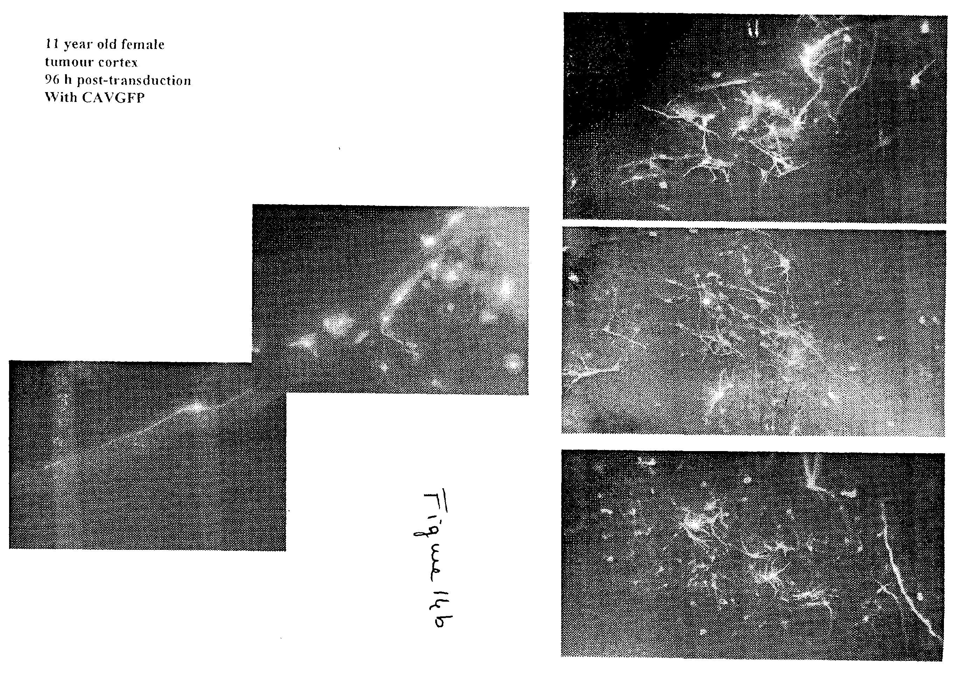

Figure 14: Human brain slices infected with CAVGFP.

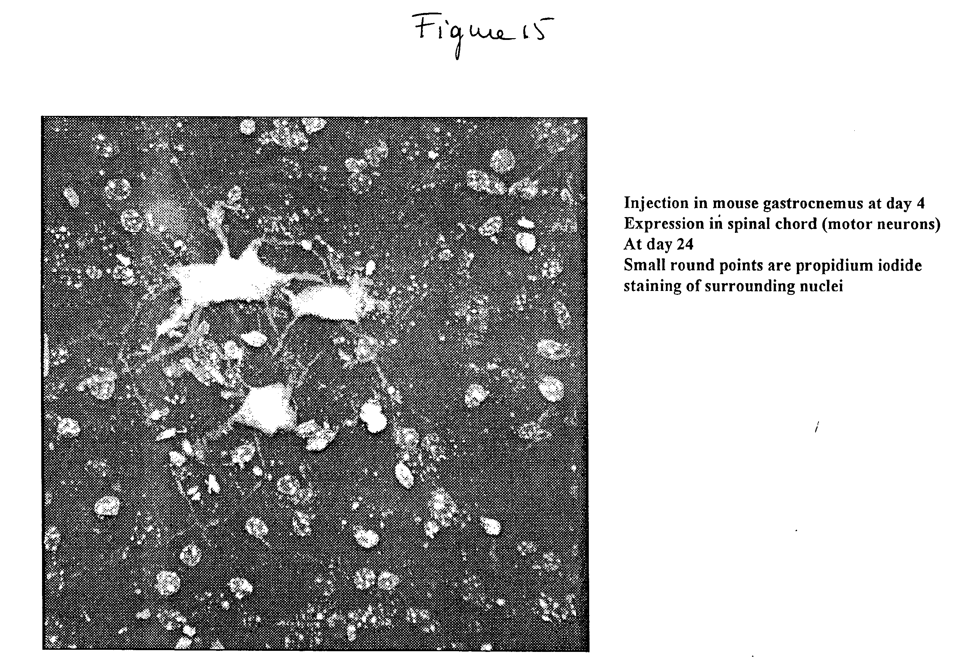

Figure 15: Section of the anterior horns of mice injected with CAVGFP.

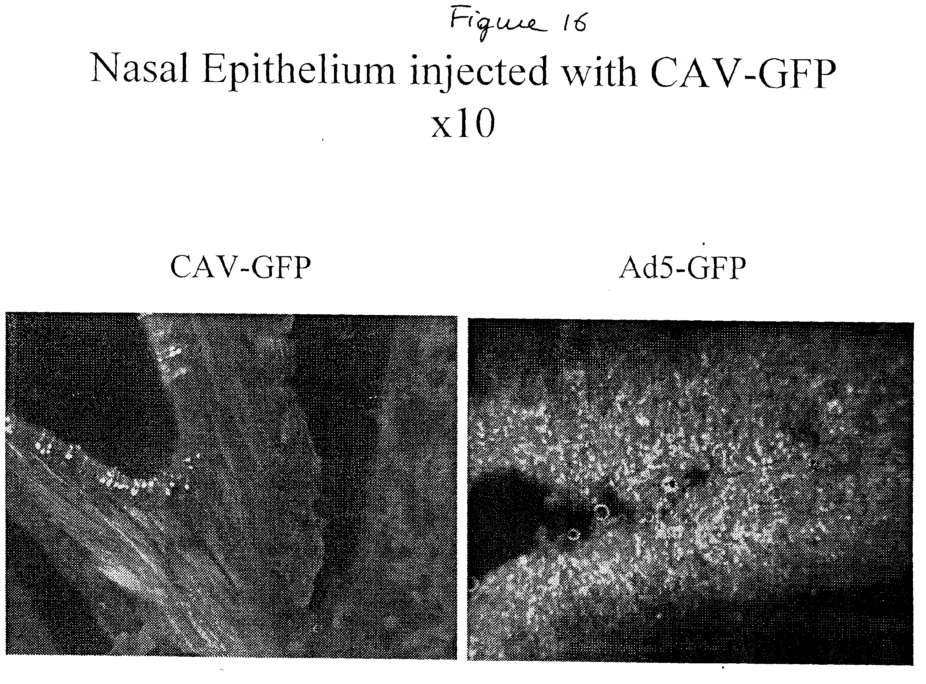

Figure 16: Ad5 versus CAV mediated transduction in the nasal cavity of the rat. CAVGFP preferentially transduced the sensory neurons while AdGFP transduced both the sensory neurons and the epithelial cells.

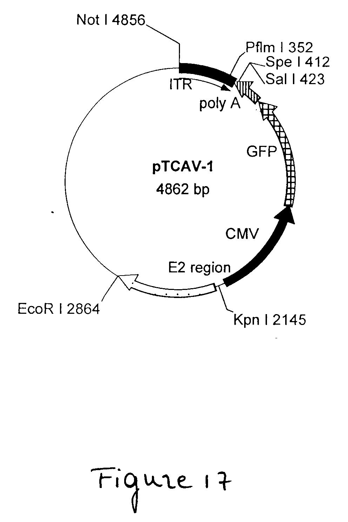

Figure 17: Schematic representation of pTCAV-1 = ptGFP (example 2) digested with EcoRI, single fragment from positions 30,629 to 2864 isolated and circularised.

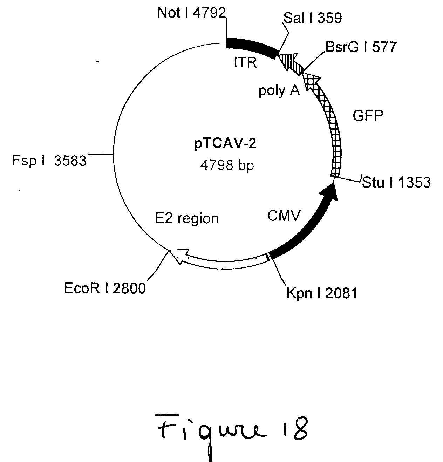

Figure 18: Schematic representation of pTCAV-2 = pTCAV-1 digested with Pfmll and Spel, ends blunted with T4 polymerase and religated.

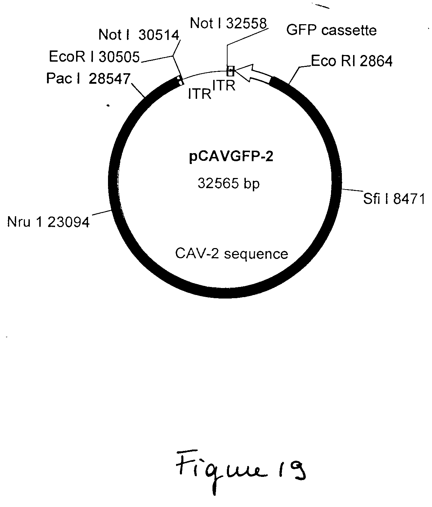

Figure 19: Schematic representation of pCAVGFP-2 = homologous recombination in BJ5183 of pTCAV-2 with pTG5412.

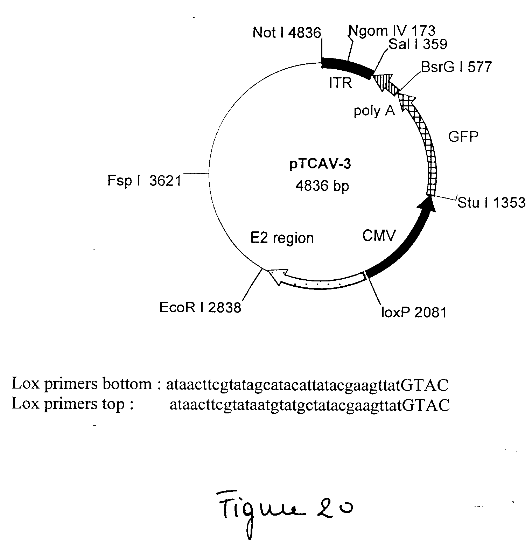

Figure 20: Schematic representation of pTCAV-3 = pTCAV-2 linearised with Kpnl and loxP primers added.

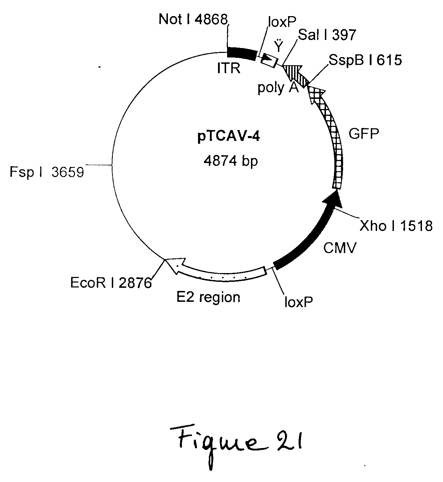

Figure 21: Schematic representation of pTCAV-4 = pTCAV3 digested with NgoM IV and added loxP from bp 174 to 216.

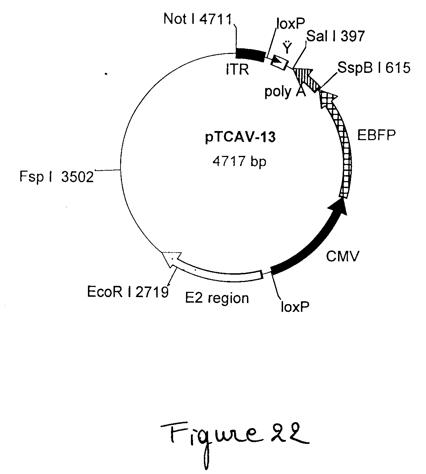

Figure 22: Schematic representation of pTCAV-13 = pTCAV-4 digested with SspBI & Xhol added SaII to SspBI from pEBFP (Clontech)

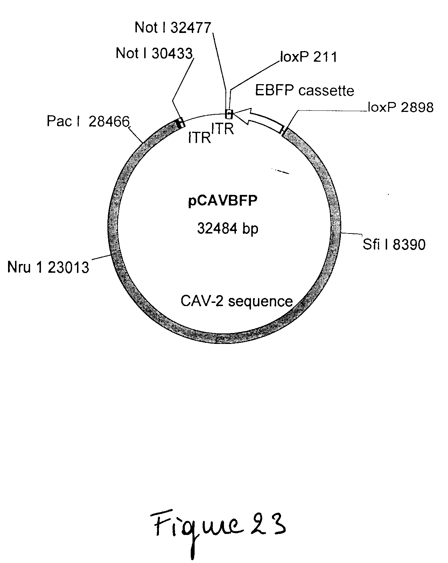

Figure 23: Schematic representation of pCAVBFP = homologous recombination between pTG5412 and pTCAV-13. Digested with NotI and transfected to make CAVBFP= Helper virus.

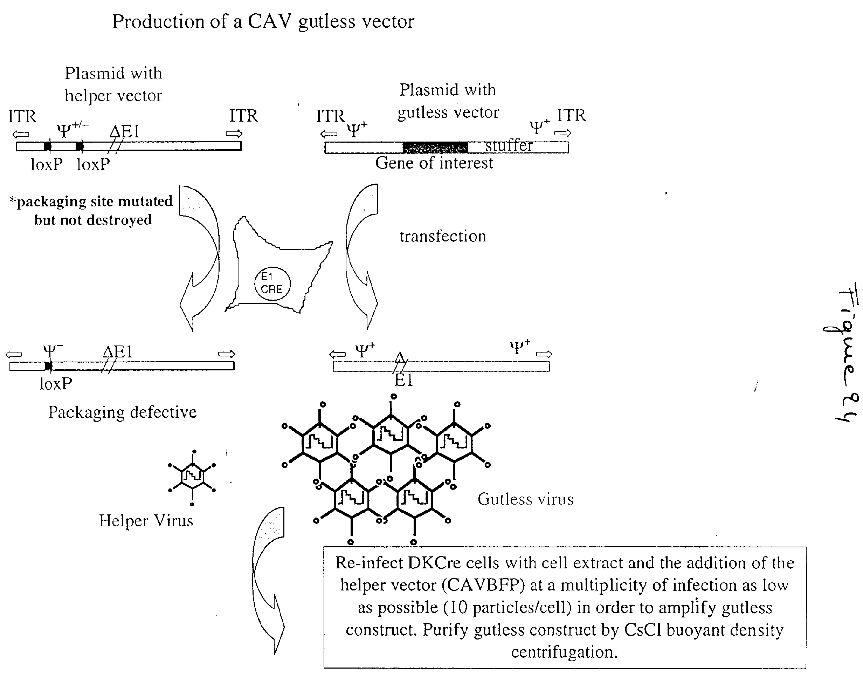

Figure 24: Schematic representation of the production protocol of CAV gutless vectors.

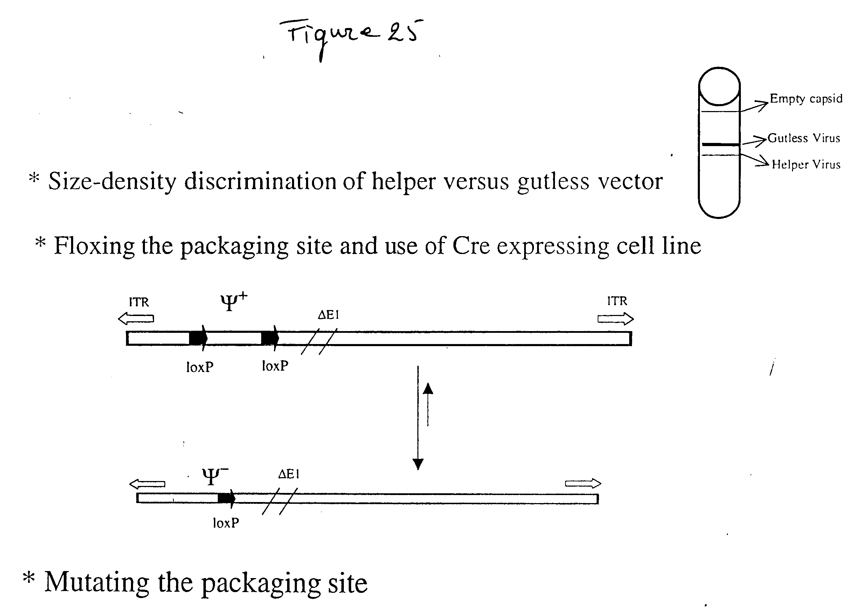

Figure 25: Strategies to reduce the contamination of gutless vector preparations by replication competent viruses.

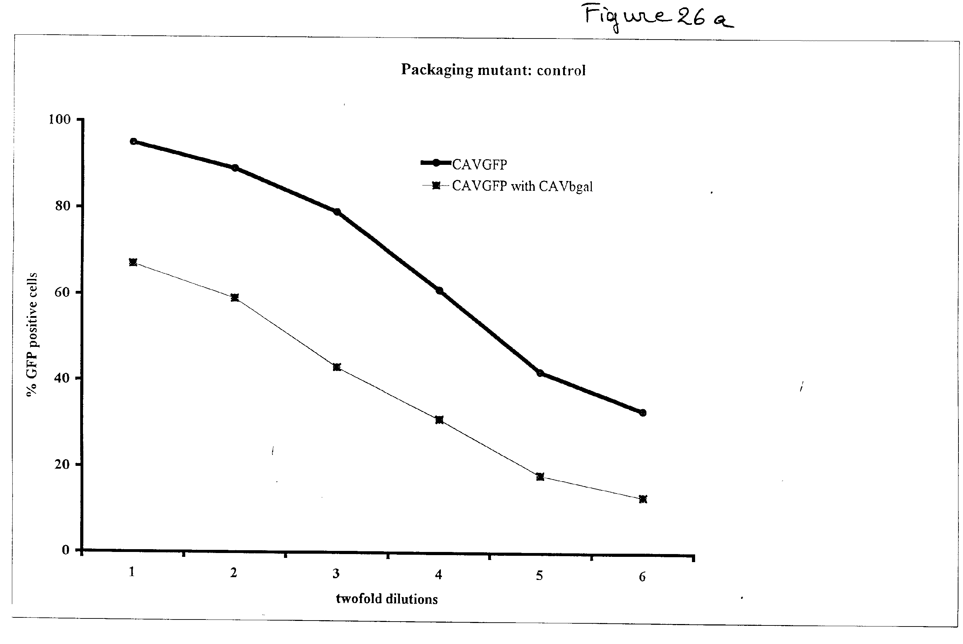

Figure 26: Results of CAV ψ sequence tests. A helper CAV vector containing a ψ mutation (Δ337) shows a packaging deficiency when in competition with the wild-type ψ sequences.

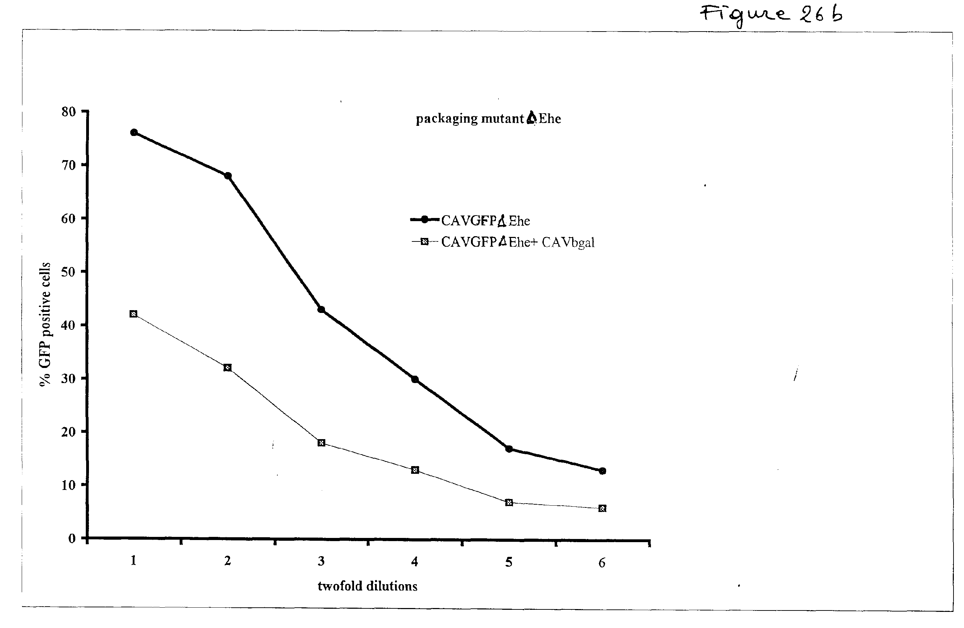

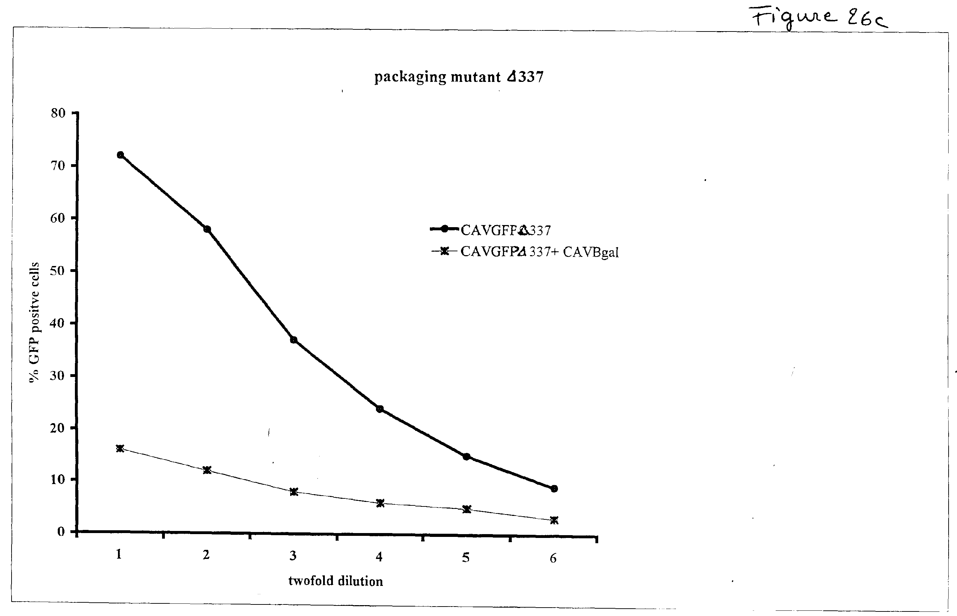

Figure 27: Schematic representation of pTCAV-6 = pTCAV-2 add K9 gutless linkers in NotI site.

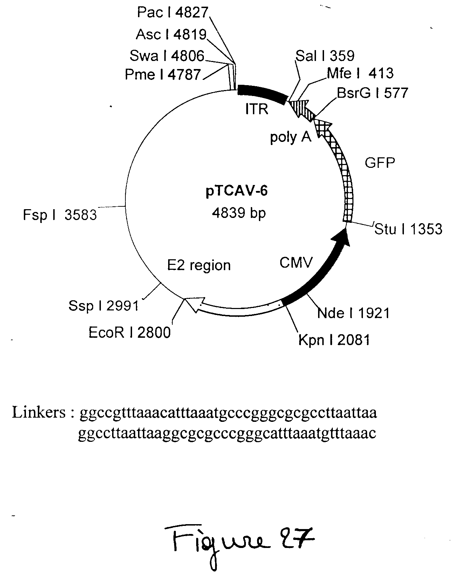

Figure 28: Schematic representation of pTCAV-11 = pTCAV-6 added MCS from pCI-Neo (Promega): Ndel to Mfel.

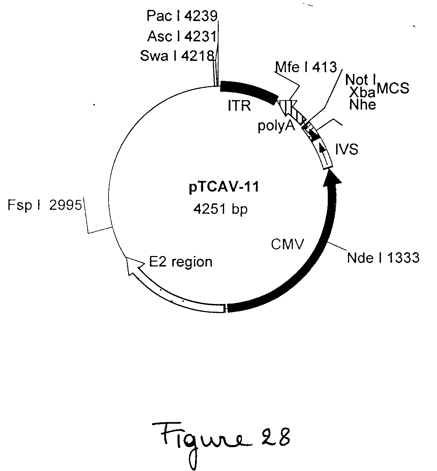

Figure 29: Schematic representation of pTCAV-12 = pTCAV-11 added "CAV link' in NotI site.

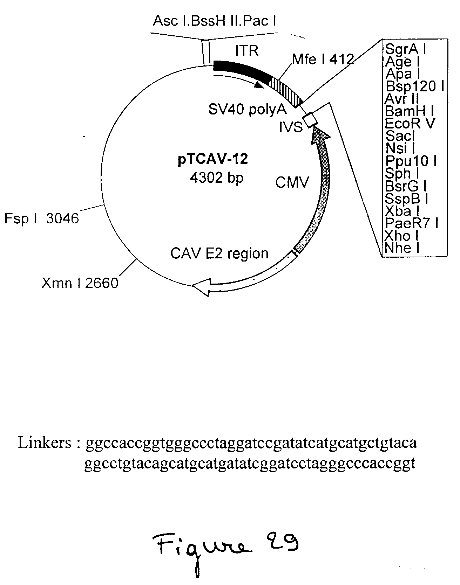

Figure 30: Schematic representation of pTCAV-7 = pTCAV-6 added ITR and 8 bp cutters from pTCAV-6 (450 bp, Pmel to Mfel) to EcoRI to Sspl sites.

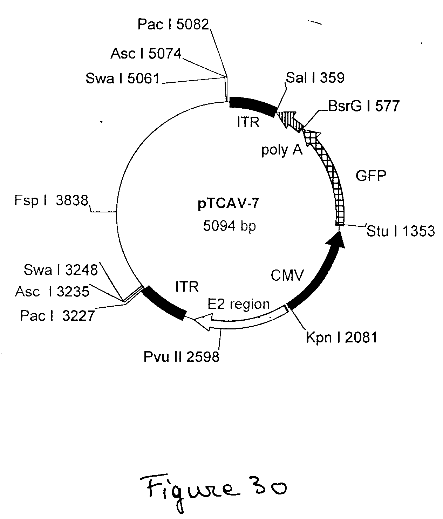

Figure 31: Schematic representation of pTCAV-8 = pTCAV-7 digested Kpnl and Pvull and religated to remove pIX.

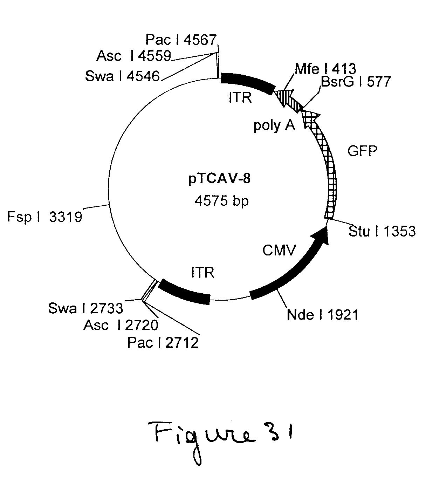

Figure 32: Schematic representation of pTCAV-9 = pTCAV-8 deleted Ndel to Mfel; added pTCAV-12a sequence from Ndel to Mfel.

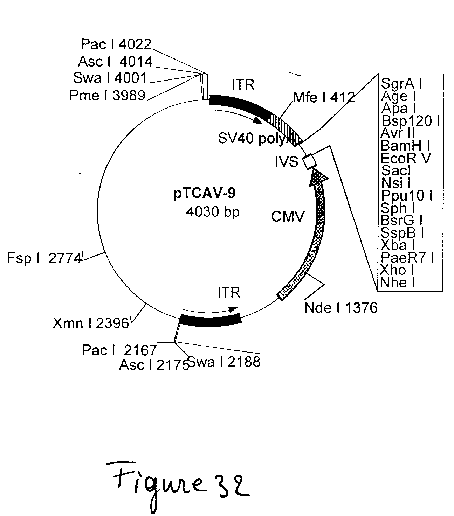

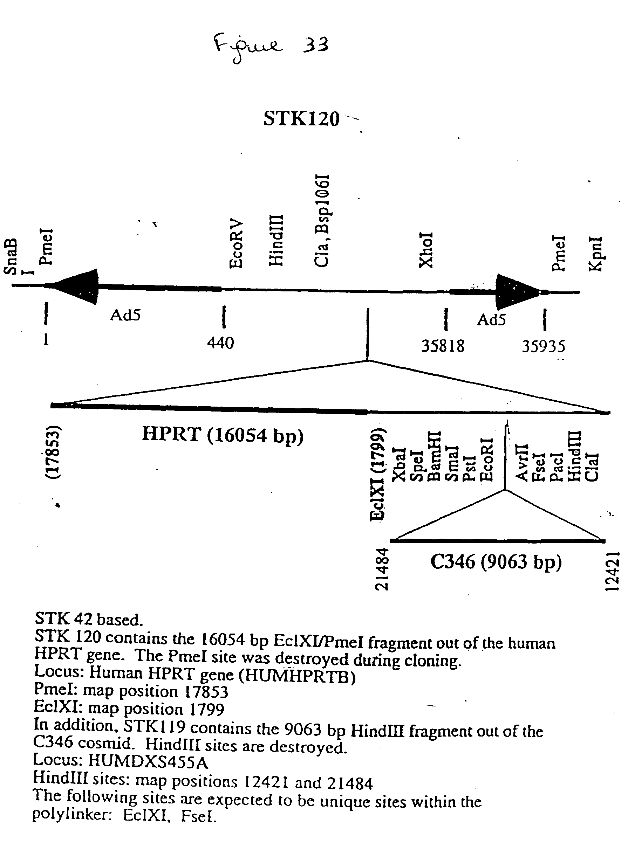

Figure 33: Schematic representation of STK120 (51).

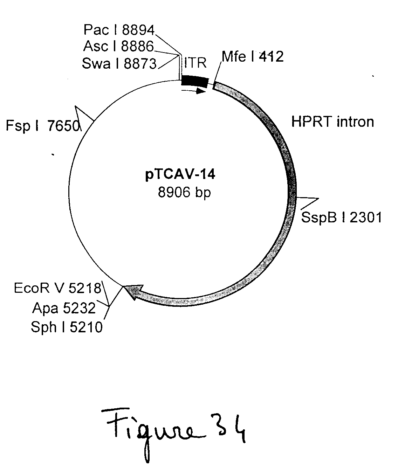

Figure 34: Schematic representation of pTCAV-14 = pTCAV-12 digested with Mfel - Sphl ; added Mfel - Sphl 4.8 kb from STK120 (51).

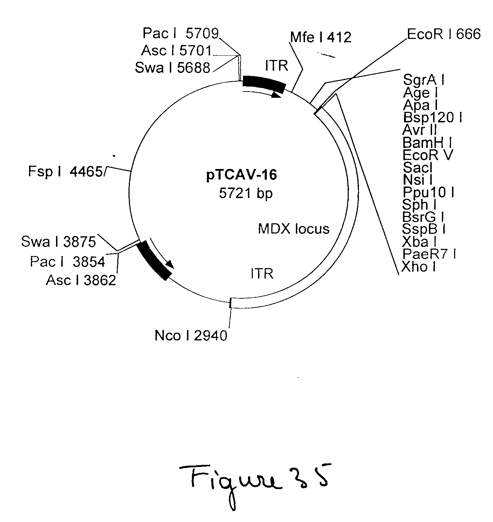

Figure 35: Schematic representation of pTCAV-16 = pTCAV-9 digested Xhol and Ncol and added : a 2269bp fragment from pSTK120 (22736 bp to 25019 bP).

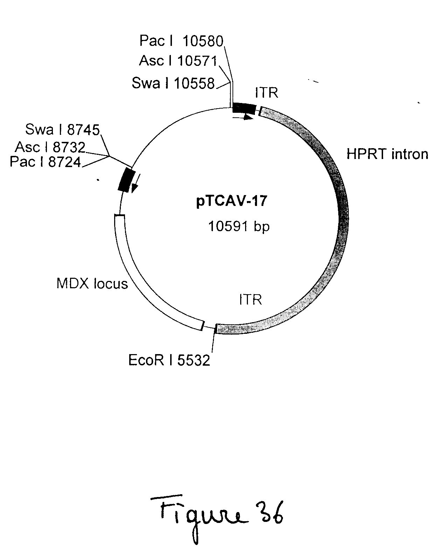

Figure 36:Schematic representation of pTCAV-17 = pTCAV-16 digested EcoRI; added Mfel to EcoRI from pTCAV-14 (4.8 bp insert).

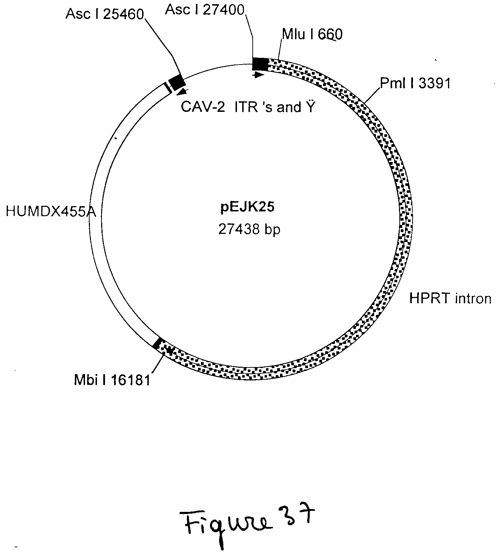

Figure 37: Schematic representation of pEJK25 = homologous recombination between pTCAV-17 linearized with EcoRI and recombined with Acll-Fsel fragment from pSTK120. Backbone plasmid for a gutless vector.

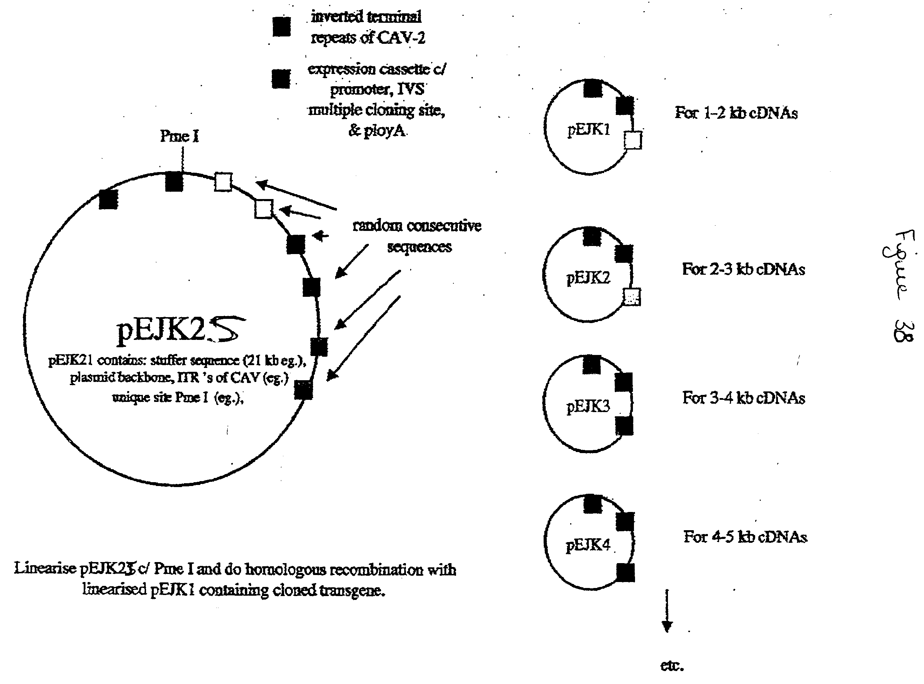

Figure 38: Strategy for the flexible generation of gutless constructs.

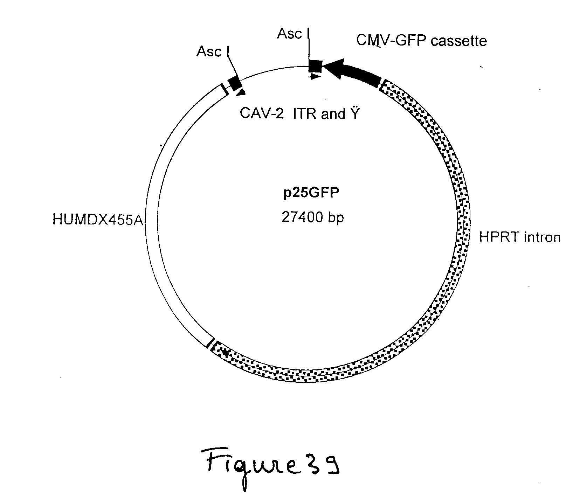

Figure 39: Schematic representation of p25GFP = pEJK25 digested with SgrAI and MIuI added 2.3 fragment from pTCAV-7 NgoM IV to Mlul. Transfer plasmid for a gutless vector carrying the GFP gene. As a proof of principle, p25GFP was also generated using pEJK1 and pEJK25. The cDNA from GFP was cloned into pEJK1 and the resulting plasmid was recombined with pEJK25 as described in figure 33 to generate p25GFP.

### Example 1 : generation of transcomplementing cell lines DK/E1-28Z and DK28Cre for E1-deleted CAV vectors

DK cells were transfected as described in Klonjkowski et al. (20) in order to generate DK/E1-28Z cells. DK/E1-28Z cells stably express neomycin and zeocin resistance genes plus the E1 region from CAV-2 Manhattan strain (nt 352-2898, Genebank seq. JO 4368). DK/E1-28Z cells have been deposited at the CNCM (Collection Nationale de Cultures de Micro-organismes) under the n°I-2292.

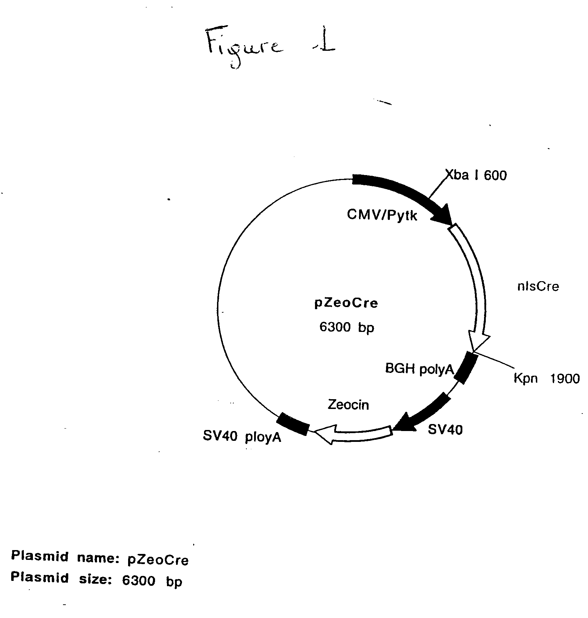

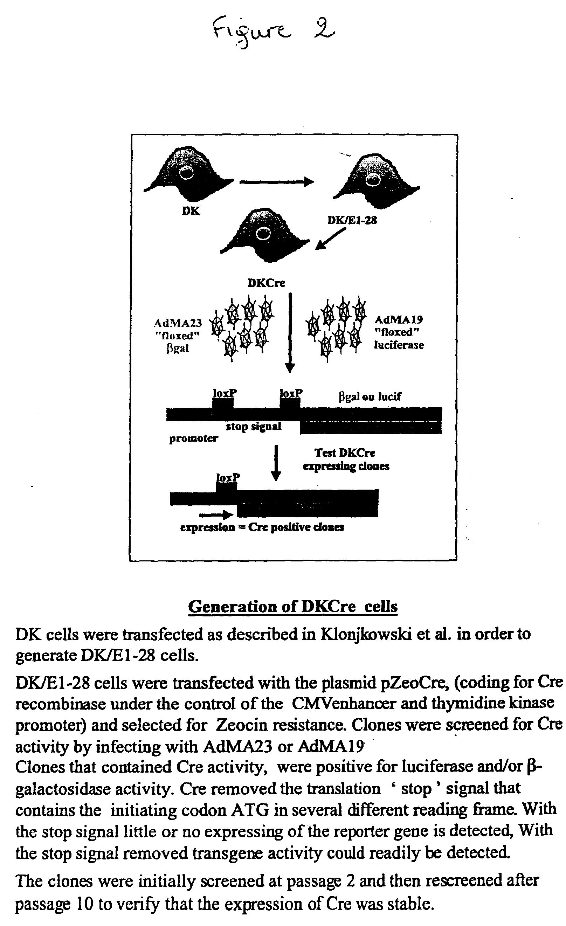

DK/E1-28 cells (20) were transfected with the plasmid pZeoCre (coding for nlsCre recombinase (46) under the control of the CMV enhancer and thymidine kinase promoter, see figure 1) and selected for Zeocin resistance. Clones were screened for Cre activity by infecting with AdMA23 or AdMA19 (ref. 50 and figure 2). Clones that contained Cre activity were positive for luciferase and/or β-galactosidase activity (table 1). Cre removed the translation "stop" signal that contains initiating codons (ATG) in several different reading frames. With the stop signal, little or no expression of the reporter gene is detected, and with the stop signal removed transgene activity could readily be detected.

The clones were initialy screened at passage 2 and then rescreened after passage 10 to verify that the expression of Cre was stable. Clone 23 was selected and will be referred to as "DK28Cre cells" hereafter. DK28Cre cells stably express neomycin and zeocin resistance genes plus the E1 region from CAV-2 Manhattan strain (nt 352-2898, Genebank seq. JO 4368) and Cre recombinase. DK28Cre cells have been deposited at the CNCM under the n° I-2293.

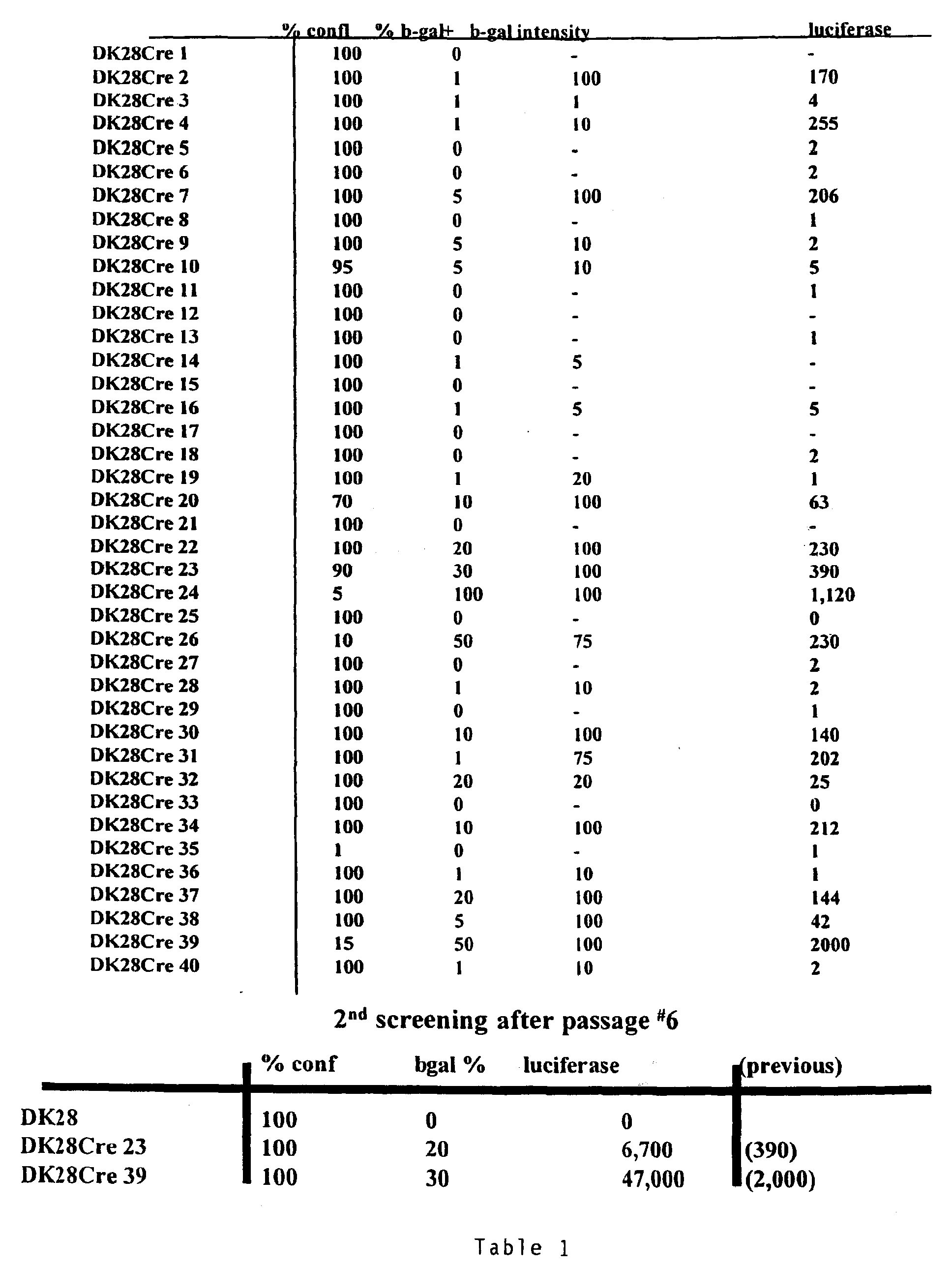

### Example 2 : canine adenovirus-mediated gene transfer

### Materials and Methods

### Cells

DK (canine kidney ATCC CRL6247), DK/E1-1 (20), DK/E1-28Z, DK28Cre, 911 (10), HT 1080 (ATCC CCL121), HeLa (ATCC CCL2) and A172 cells (ATCC CRL 1620) were grown in DMEM (GIBCO), 10% foetal calf serum (BioWhittaker) and 2 mM glutamine (GIBCO). DK/E1-1, DK/E1-28Z and DK28Cre contain the CAV-2 E1 region stably integrated in the genome with the E1A region under the control of the CMV promoter and the E1B region under the control of its own promoter. In order to try to increase vector production, we tested two DK/E1-28Z subclones for their ability to amplify the CAV vectors. One of the two, DK28Cre cells, gave a homogenous infection pattern and a higher yield. DK/E1-1, DK/E1-28Z and DK28Cre are derived from DK cells, an immortalised line.

### Plasmids and viruses

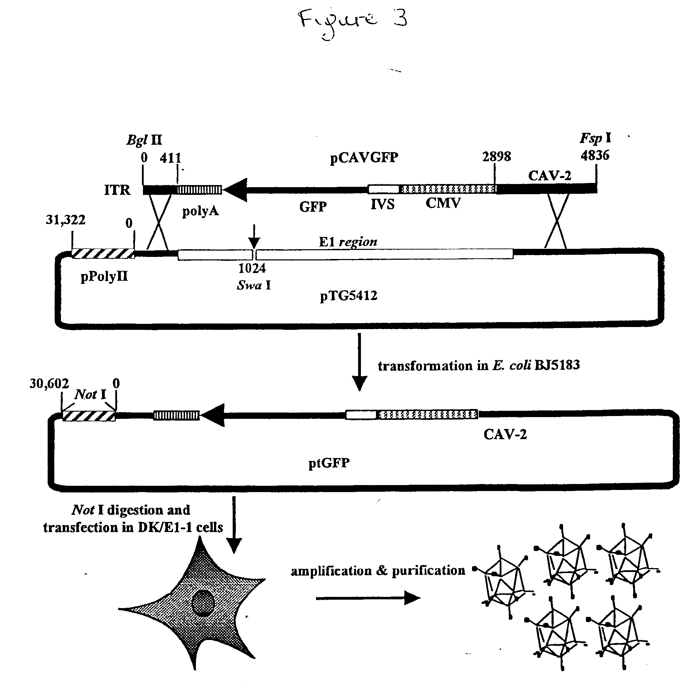

DNA preparations, restriction enzyme digests and Southern blot analysis were performed under standard conditions (2). The construction of the pre-transfer and transfer plasmids, pCAVGFP and ptGFP, is represented in figure 3. Briefly, pCAVGFP contains the first 411 bp of the left end of CAV-2 and a GFP expression cassette containing a CMV early region enhancer/promoter, a SV40 intron with splice donor and acceptor sites (IVS), the humanised red-shifted version of the *Aequorea victoria* green fluorescent protein (EGFP, Clontech) and an SV40 polyadenylation site followed by bp 2898-5298 of CAV-2 cloned in pSP73 (Promega). The expression cassette is transcribed from right to left in plasmids and GFP-expressing CAV vectors. pTG5412 contains the CAV-2 genome (strain Toronto A 26/61 Genbank, 477082) flanked by *Not* I sites cloned in pPolyll. pTG5412 was generated using the same strategy as that used to generate pTG3602 (6) except *Not* 1 linkers were used instead of *Pac* 1.

ptGFP, and other transfer plasmids used to produce vectors, were generated by *in vivo* homologous recombination in *Escherichia coli* strain BJ5183 according to Chartier *et al.* (6) using *Swa* I-linearised pTG5412 and a fragment containing the inverted terminal repeat, the GFP expression cassette and the CAV-2 E2B regions. CAVGFPΔE1A, is deleted in the CAV-2 genome from bp 411 to 1024. ptGFP, and therefore the virus CAVGFP, are deleted in the CAV-2 genome from bp 411 to 2898. CAV vectors were partially sequenced directly from low molecular weight DNA preparations from infected DK/E1-28Z cells to verify their integrity. AdGFP is a first generation E1, E3-deleted human adenovirus 5 vector containing a GFP expression cassette similar to the one in the CAV vectors except a) that the transcription unit is oriented left to right and b) contains *Not* I sites flanking the transgene.

### CAV vector preparation and purification

Described is the preparation of CAVGFP, other CAV vectors were prepared in similar fashion. Transfections in DK/E1-28Z cells were with 5 pg of *Not* I-digested ptGFP and 20 µl of LipofectAmine (GIBCO) in 6 well plates containing approximately 10⁶ cells. DK/E1-28Z cells were collected when a cytopathic effect was detected 1 to 2 weeks post-transfection and the vector freed from the cells by 4 freeze/thaw cycles and centrifugation to remove cellular debris. The cleared lysate was incubated with a fresh monolayer of DK/E1-28Z cells and collected 48 hours post-infection. This was repeated 4-5 times until a prestock of ten 10 cm dishes showed a complete cytopathic effect 48 hour post-infection. This "prestock" was used to infect fifty 15 cm plates of DK/E1-28Z cells. Forty hours post-infection the cells were collected, the vector freed by 4 freeze/thaw cycles. Approximately 7 ml of cleared lysate was layered on a step CsCI gradient of 1.4 gm/ml, and 1.25 gm/ml (2.5 ml each layer) and centrifuged for 90 minutes using a Beckman SW 41 rotor at 35,000 rpm. The CAVGFP band was removed and further purified on a CsCI isopycnic gradient at a density of 1.32 gm/ml (versus the 1.34 gm/ml used for human adenovirus vectors) gradient for 18 hours, using the same speed and rotor. Both centrifuge runs were at 18°C. CAV vectors banded at a density of ~1.22 gm/ml. CsCI was removed using PD-10 columns (Pharmacia) and the virus stored in PBS containing 10% glycerol.

### Titration of CA V-2, AdGFP and CA V vectors

Vector concentration as determined by OD₂₆₀ was done using two dilutions of two aliquots of each virus/vector stock as described (30). The inventors have assayed the particle to transduction unit ratio in the most sensitive assay they could develop. DK28Cre cells are the largest of the three cell types tested (DK, DK/E1-28Z and DK28Cre), are the most sensitive to CAVGFPΔinfection and give a homogenous infection pattern. For the transduction unit titration of CAVGFPΔE1A and CAVGFP, DK/E1-28Z or DK28Cre cells were seeded in 12 well plates and infected overnight with gentle rocking with 2-fold dilutions beginning with 1.25 x 10⁶ viral particles/well. Twenty-four hours post-infection the cells were analysed by flow cytometry (FACSCalibur, Becton Dickinson), the percentage of GFP-positive cells was determined and used to calculate the particle to transduction unit ratio, (input viral particles) (GFP-positive cells)⁻¹. Mock-infected cells and cells infected with CAVΔE1 were used as negative controls and no background fluorescence was detected. AdGFP was similarly titrated on 911 cells, which were used because they are 3-fold more sensitive to human adenovirus vectors, when compared to 293 cells (10). Plaque forming units of AdGFP and CAV-2 were performed as follows: 0.5 ml of 10-fold dilutions of virus/vector was incubated with a confluent monolayer of 911 or DK cells in a 30 mm well overnight before a layer of agarose was used to cover the cells. The titre was determined 6 and 14 days post-infection, respectively.

In order to determine if there were background from green fluorescent protein transfer (pseudo-transduction), 12 well plates containing a confluent monolayer of DK28Cre cells were infected at 4°C with CAVGFP for 4 and 6 hours at an input ratio of approximately 10³ particles/cell. The plate was rocked continuously, transferred to 37°C for 30 minutes, the cells trypsinised and an aliquot was assayed by flow cytometry. The remaining cells were returned to 37°WITH6% CO₂ and analysed by flow cytometry 24 hours post-infection.

### RCA assays

High titre stocks of Ad vectors produced on 293 cells are often contaminated with replication competent Ads (RCA's) which are generated by homologous recombination of the vector and the E1 region, which is stably integrated in the cell line. Repeat amplification of the vector favours the probability of generation of RCA's. DNA from DK/E1-28 cells, the E1-transcomplementing cell line, was digested with 4 restriction enzymes that cut once in the stably integrated E1 expression cassette and Southern blot analysis (2) demonstrated that there is a single copy of the CAV-2 E1 region.

2.5 x 10¹⁰ particles of CAVβgal (divided equally into nine, 15 cm dishes) and 5 x 10¹⁰ particles CAVGFP (17 dishes), from two separate stocks, were assayed. Each dish, containing 1.3 x 108 DK cells/plate, was incubated overnight with 2.7-3.0 x 10⁹ particles of CAVGFP or CAVβgal/plate (maximum of 23 particles/cell), with gentle rocking in a humidified chamber at 37°C. The plates were removed from the shaker and placed in an incubator (6% CO₂/37°C) for 5-6 days before the cells were collected, and the cleared lysate used to inoculate a second plate containing 5 x 10⁷ DK cells. The cleared lysate was removed from the cells 1-2 days later, fresh media was added and the cells collected 3-4 days later. This was repeated until the positive controls (two 15 cm plates containing DK cells infected with 3.0 x 10⁹ particles of CAVGFP, spiked with 10² particles of CAV-2, and amplified as above) showed an extensive CAV-2 induced cytopathic effect (3 passages). The cultures transduced with CAVGFP and CAVpgal were passed an additional time, and still showed no sign of cytopathic effect.

### Transduction of human cells: CAVGFP vs. AdGFP

To compare the infection efficiency on human cell lines, identical 24 well plates containing monolayers of HeLa, HT 1080 or A172 cells (approximately 10⁶ cells/well) were infected with 5-fold dilutions of CAVGFP or AdGFP starting with 4.3 x 10⁸ and 1 x 10⁹ particles/well, respectively. The cells were collected 48 hours post-transduction and assayed for GFP expression by flow cytometry. The number of particles needed to generate 10% GFP-positive cells was calculated. Ten percent was in the range of one transduction unit/GFP-positive cell.

### In vivo use of CAVβgal, CAVGFP and AdGFP

All mice were treated according the rules governing animal care for the European Community. Eight week-old BALB/c mice (n = 10) were lightly anaesthetised using halothane (Belamont) and 10¹¹ particles, diluted in PBS (100 µl total volume), were delivered intranasally. Mice were sacrificed on day 3, 4 or 21 and the lungs were recovered following perfusion with 2% paraformaldehyde and embedded in O.C.T. (Tissue-Tek). GFP expression was detected using a Zeiss Axiovert fluorescent microscope with an EGFP filter (485-507 nm) at an original magnification of 10X.

### Neutralising adenovirus antibodies

Fifty samples of whole blood were purchased from the Centre Transfusion de Rungis, (Rungis, France). Serum was separated and complement-inactivated at 56°C for 30 minutes. Ten microliters of serum was mixed with 100 µl of media containing 5 x 10⁷ vector particles (CAVGFP or AdGFP) for 1 hour at room temperature, prior to incubation with 911 cells. The cells were tested for GFP expression by flow cytometry 24 hours post-infection. Each sample was done in duplicate and repeated. Results similar to AdGFP were obtained with an adenovirus type 2 vector expressing GFP (not shown).

### Results

### Isolation of CAVGFP

Four adenovirus vectors derived from CAV-2 are described here: CAVGFPΔE1a and CAVGFP, which harbour the gene encoding GFP, CAVβgal, encoding nuclear localised β-galactosidase, and CAVΔE1, which contains a null expression cassette (see Material and Methods and summary in Table 2). Figure 3 shows a diagram of the plasmid used to generate CAVGFP. *Not I*-digested ptGFP, which places the ITR's at the extremities of the DNA fragment and allows vector replication, was transfected into DK/E1-1 cells. In order to further characterise DK/E1-1 and DK/E1-28Z cells, the CAV-2 E1 expression cassette was amplified by PCR from total genomic DNA and the PCR product sequenced. The sequence was identical to the transfected plasmid and the published CAV-2 sequence.

Because they were using GFP as the transgene, the inventors were able to monitor the propagation of the vector post-transfection. One to two weeks later, the cells were collected and the cleared lysate used to amplify the vector. CAVGFP and CAVGFPΔEIa DNA were extracted from CsCI purified vector stocks and digested with *EcoR I* (Figure 4a). All digests gave the anticipated pattern for each vector when compared to their respective transfer plasmid. No contaminating bands were detectable by ethidium bromide staining in any restriction enzyme digests (n = 6). These results demonstrated that these CAV vectors are free of gross rearrangements, deletions or insertions. CAVβgal DNA was also analysed by restriction enzyme digests (n = 5) and no extraneous bands were detected (not shown). In order to further verify the integrity of CAVGFP, the digestions were assayed by Southern blot analysis using PCR generated fragments from the CAV-2 E1 region (bp 458-936, Figure 4b) or the GFP cDNA (Figure 4c) as the radiolabelled probe. No signal was found in pTG5412 for the GFP-derived probe, as expected, while the predicted size fragments, 2.89 and 4.75 kb (Figure 4d), were detected in the CAV vectors. The E1 region probe hybridised to the 3.6 kb band in pTG5412 as expected, but failed to hybridise specifically to CAV vector sequences. Southern blot analyses confirmed that the vectors did not acquire E1A-derived sequences during isolation or amplification.

### Vector preparation, titration and purity

Stocks of CAVGFP were generated containing 2.3 x 10¹² particles/ml, with a particle to transduction unit ratio of less than 3:1. CAVGFP vector yield was ~10⁴ particles/cell, similar to the ratio found when using PERC.6 cells to produce first generation human adenovirus vectors (11). Due to the exceptionally low particle to transduction unit ratio in CAVGFP, the inventors asked if the capsid contained GFP and, therefore, they were detecting protein transfer instead of gene transfer. In order to assay this, purified CAVGFP was incubated with DK28Cre cells at 4°C to allow attachment of the vector to the cellular receptor. The cells were placed at 37°C to induce internalisation of the vector and analysed by flow cytometry. Subsequently, the cells were returned to the incubator, and assayed by flow cytometry 24 hours post-infection. No GFP-positive cells were detected following the attachment/internalisation step, while 34% of the cells were GFP-positive 24 hours post-infection, demonstrating that this assay was detecting gene transfer and not protein transfer. CAVGFP is 97.7% of the size of the wild type CAV-2 genome (31,322 bp), CAVΔE1 (not shown) is 95.2%, and CAVβgal is 105.7%. Stocks of CAVβgal were generated at a concentration of 5.2 x 10¹² particles/ml and a particle to transduction unit ratio of approximately 10:1.

With human adenovirus vectors the generation of replication-competent adenoviruses (RCA) and E1 region containing particles during stock preparation is a significant clinical concern. With CAV vectors the risks associated with RCA's are diminished, if not completely eliminated, because CAV-2 does not propagate in human cells. However, the E1 region of many adenoviruses encodes potentially oncogenic proteins that can transform or immortalise cells *in vitro* and *in vivo* (36) and therefore must be deleted from an adenovirus vector if it is to be used in patients. The inventors generated E1-transcomplementing cells to propagate these vectors and designed the cell line in order to try to reduce the likelihood of generating replication-competent CAV-2. CAVGFP and CAVβgal stocks were tested for the presence of replication-competent CAV-2 using a serial amplification on permissive cells (DK cells). The sensitivity of this assay was 1-2 plaque forming units/5 x 10¹⁰ particles, as 100 particles of CAV-2 (1 pfu/66 particles) were used to spike 3 x 10⁹ CAVGFP particles/plate as a positive control. They were unable to detect a CAV-2 induced cytopathic effect, demonstrating the lack of replication-competent CAV-2 in 5 x 10¹⁰ particles of CAVGFP (2.5 x 10¹⁰ particles of each stock) and 2.5 x 10¹⁰ particles of CAVβgal.

### Transduction of human-derived cells: CAVGFP versus AdGFP

Inventors' team demonstrated previously using a qualitative assay that a CAV vector derived from the Manhattan strain of CAV-2 could transduce human-derived cells (20). However, it was impossible to determine the efficacy of transduction because the "vector stock" contained significant amounts of CAV-2 (virus/vector ratio was >10,000:1). In order to determine the quantitative transduction efficiency using the CAV vector described here (Toronto strain), three human cell lines, HT 1080, HeLa and A172 cells, which are derived from different cell lineage (osteosarcoma, cervical carcinoma and glioblastoma) were quantitatively assayed for their transducibility. Multiwell plates, containing an equal number of each cell type, were incubated with a serial dilution of CAVGFP and AdGFP. Forty-eight hours post-transduction the cells were assayed for transgene expression by flow cytometry. Figure 5 shows the particles to cell ratio needed to generate 10% GFP-positive cells/well. In each cell line, CAVGFP was 5-10 fold more efficient (lower number of particles needed) than AdGFP when compared as particle/cell ratio. However, we have found that the quality of adenovirus vector preparations can vary significantly. If the comparison between CAVGFP and AdGFP is plotted as transduction units/cell versus percent GFP⁺ cells/well, the transduction efficiency of AdGFP in HeLa cells is slightly greater than that of CAVGFP (Figure 5b).

### In vivo use of CAV vectors and comparison to AdGFP

An *in vivo* study was used to assay the utility of CAV vectors. 10¹¹ particles of CAVβgal and CAVGFP were delivered intranasally in 8 week-old BALB/c mice. Nuclear-localised β-galactosidase activity was detected throughout the proximal and distal airways and in the alveoli. In some instances where expression was detected in the alveoli, thickening of the cell walls was visible (not shown) suggesting cellular infiltration, and 21 days post-transduction we were unable to detect GFP expression (n=3). CAVGFP was able to transduce greater than 65% of a given distal airway was GFP-positive (d and e). Comparison of the transduction efficiency (e versus g) of CAVGFP versus AdGFP (f and g) demonstrates that CAV vectors can be as efficient *in vivo* as those derived from human adenoviruses.

### Pre-existing humoral immunity

The majority of individuals has been exposed repeatedly to adenoviruses and, not surprisingly, have detectable neutralising adenovirus antibodies. Serum from a random healthy cohort (n=50) was tested for its ability to neutralise AdGFP and CAVGFP transduction. Figure 7 demonstrates that in most cases (26/50), as little as 10 µl of human serum contains sufficient amounts of neutralising Ad 5 (as well as Ad-2, not shown) antibodies to rapidly and completely inactivate 5 x 10⁷ AdGFP particles. These sera rarely (1/50) contain detectable neutralising CAV-2 antibodies. *In vivo,* airway epithelia transports both IgG and IgA to the thin layer of liquid that covers the apical surface of the epithelia, and can prevent adenovirus infection. These data are particularly significant because if one can not circumvent this initial barrier for adenovirus-mediated gene transfer, use of human adenovirus vectors becomes limited. These CAV vectors, and importantly more advanced versions, are not inhibited at this stage.

**Table 2**

| Summary of virus and vectors | | | | |
|---|---|---|---|---|
| Virus/vector | particles/ml | part/t.u.¹ | % wt² | RCA³ |
| AdGFP | 1.3 - 4 x 10¹² | 10 - 26:1 | 88.1 | n.t. |
| CAV-2 | 1.9 x 10¹² | n.a. | 100 | n.a. |
| CAVGFP | 0.3-2.3 x 10¹² | 3 - 7:1 | 97.7 | No^{a} |
| CAVΔE1 | 3.7 x 10¹¹ | n.a. | 95.2 | n.t. |
| CAVβgal | 2.6-5.2 x 10¹² | 10:1 | 105.7 | No^{b} |
| | | | | |
| 25GFP | 2 x 10⁹ | n.t. | 80 | n.t. |

| | | | | |
|---|---|---|---|---|
| ¹particle to transduction unit ratio in producer cell line | | | | |
| ² percentage of the wild type genome length, Ad5 or CAV-2 | | | | |
| ³less than 1-2 replication competent adenovirus particles/^{a}5 x 10¹⁰ and ^{b}2.5 x 10¹⁰ particles n.t.: not tested, n.a: not applicable | | | | |

### Discussion

The inventors have generated a system to produce canine adenovirus vectors for gene transfer. Several reasons seem plausible for their ability to generate replication-competent-free CAV vectors using this strategy versus their previous attempts (20). The previous strategy (transfection of two linear fragment of DNA in DK/E1 cells and hope for homologous recombination to generate a recombinant vector) was similar to that used to generate first generation human adenovirus vectors (23). Initially, DK cells and their derivatives are difficult to transfect, normally lower than 15% efficiency. Secondly, DK cells may also be less efficient at homologous recombination than 293 cells. Finally, the Manhattan strain of CAV-2 is unstable - it is able to generate at least 23 repeats of~120-150 bp in the right inverted terminal repeat (unpublished data and (13)). All of these factors may have prevented from isolating pure vectors. Using the strategy described here, the inventors have eliminated the need for high transfection efficiency, homologous recombination in the cell line, and the presence of the unstable sequence in the inverted terminal repeat.

The stable packaging capacity of the human adenovirus 5 vectors was determined to be a minimum of 75% (31) and maximum of 105% of the wild type genome (4). The size of the GFP-expressing CAV vectors are within this range, while CAVβgal is slightly larger (see Table 2) and appears to be stable. Xu *et al.* (39) reported the creation of an ovine adenovirus vector that is 114% of the wild type genome, demonstrating that the cloning capacity of this and other adenoviruses may not mimic that of the adenovirus 5 vectors. Stocks of CAVGFP have a particle to transduction unit ratio as low as 3:1, while CAVβgal stocks have a particle to transduction unit ratio of approximately 10:1. Mittereder et *al.* (30) have carefully detailed the physical and biological parameters used to titre adenovirus vectors. Taking into account their work, the particles to transduction unit ratio may be an underestimation of the true titre, due to undetectable transgene expression from transduction occurring later during the incubation period. More CAV vectors will need to be generated to determine if the low particle to transduction unit ratio in these initial stocks is a general trend, an exception in these cases or due to a more sensitive quantification assay.

All the CAV vectors, including E1A-deleted, are replication-defective in DK, MDCK and more significantly 911 cells. This demonstrates that there is an undetectable level of transcomplementation of the adenovirus 5 E1-derived proteins in these cells for CAV vector propagation. Although contamination of CAV vector stocks with RCA is certainly undesirable, it is significantly less dangerous than contaminating replication-competent human adenovirus that may be below the level of detection. The El-transcomplementing cell lines described here do not contain the CAV-2 inverted terminal repeat or the packaging signal found at the left end of the CAV-2 genome, but do contain a 55 bp of overlap in the E1A promoter with the vectors described here. The inventors have generated other CAV vectors that do not contain an overlap in this region (example 5) and all subsequent CAV vectors will not be able to generate RCA's via the *in vivo* mechanism characterised by Hehir *et al.* (17).

As mentioned previously, adenovirus infections can be dangerous in infants and immuno-compromised patients. Replication-competent adenoviruses have been found in patients' tonsils, adenoids and intestine and patients can continue to shed adenovirus intermittently for many months after a successful humoral response (18). Immuno-tolerisation against a ubiquitous, potentially lethal virus may expose patient to unacceptable risks. If immuno-tolerisation is an unavoidable requirement to adenovirus-mediated therapy, our data demonstrate that it may be contemplated using CAV-2-derived vectors. Furthermore, reducing the viral input load due to a lower particle to transduction unit ratio (Table 2) will diminish the induced immune response to the virus capsid.

The population as a whole is being exposed repeatedly to wild type adenoviruses and the clinically relevant data presented here demonstrates that a significant proportion (98%) of this cohort has not generated neutralising CAV-2 (Toronto strain) antibodies. Using inbred rodent strains to assay induced humoral or cellular immunity to a human adenovirus vector, followed by a challenge with CAV vectors, may also allow one to detect anti-human adenovirus antibodies that opsonize rather than neutralise CAV vectors. Crossspecie barriers to adenovirus infections exist not because of the lack of infectibility, but due, at least in part, to the incompatibility of viral and cellular factors. For example, human adenoviruses grow poorly in monkey cells due to the inefficient transport or processing of the E4 and late region primary transcripts (34, 35).

The inventors demonstrated that CAV vectors could efficiently transduce human cells, and that the transduction efficiency was at least equal to that of an adenovirus 5 vector carrying the same expression cassette. Analysis of the efficacy of various human adenovirus serotypes suggest that adenovirus 2 and 5 may not be the optimal adenovirus serotypes for gene transfer in many tissues, and therefore the comparison using CAV vectors is useful, but not allencompassing. It will be interesting to determine if the receptor used by CAV-2 is the same as that used by Ad5 (3). However, the future of viral vectors will be with tissue-specific transduction and, in the case of adenovirus vectors, the fibre knob will be modified accordingly (38). Alternatively, efficient *in vivo* fibreindependent transduction using adenovirus vector/calcium phosphate precipitates (12) or polycations (19), which increase the transduction efficiency by 10-100-fold, may be applicable.

The CAV-2 fibre appears to be a trimer as determined by protein sequence analysis (1) and comparison to human adenovirus 2, 40 and 41. The Toronto strain of CAV-2 has been shown to preferentially infect the upper respiratory tract of dogs but has also been found in the faeces of infected animals (15). This tropism has been suggested to be due not only to the expression of the receptor, but potentially to the role of the E3 region (25). We tested CAV vectors were tested via intranasal delivery in BALB/c mice and effective transduction was detected in proximal and distal airway cells, as well as in the alveoli. We did not detect a site preference in the lung, and the disappearance of β-galactosidase activity and GFP expression suggested that there would be little difference between E1-deleted CAV and human adenovirus vectors with respect to the inevitable immune response.

### Example 3 : Use of viral vectors derived from canine adenovirus to confer a gain of function specifically on neurons in vivo.

Here is described the neuronal tropism and the entry into neuronal cells, by interaction with neuritic or axonal processes, of CAV vectors. These properties open up the possibility of achieving the genetic modification of neurons, the specific targeting of a therapeutic gene throughout the whole of certain given neuro-anatomical structures as well as to particular neuronal populations of central grey nuclei.

### Experimental procedure:

### Animals and surgical procedure:

Under pentobarbital anesthesia, male Sprague-Dawley rats received intracerebral injection of 10⁸ particles of CAVGFP or AdGFP. The preparation was injected over 50 mn, and afterwards the cannula was left in place for additional 2 mn (See Example 2. for the description of vector design). These intracerebral injections were performed in : Striatum, Hippocampus, somatosensory cortex, motor cortex, Globus Pallidus, Thalamus, according to the stereotaxic coordinates given by Paxinos and Watson, *(The rat brain,* in stereotaxic coordinates, Academic Press).

### Perfusion and immunohistochemistry:

Rats were lethally anesthetized and perfusion fixed (4% paraformaldehyde). Brains were post-fixed and dehydrated in 20% sucrose/0.1 phosphate buffer. Coronal sections were cut (20 µm), and free-flotting sections were processed for immunohistochemistry according to the manufactured protocol for either GFAP, NeuN and TH antibodies.

Cyanine3 conjugated vectors were used according to the manufactured protocol (CY3 linked, Amersham).

### Results:

### - Neuronal specificity:

The neuro-anatomical study of rat brains injected with this vector in different structures (striatum, hippocampus or cortex), indicated a strong neuronal tropism (fig. 8). Control animals injected with vectors derived from the human adenovirus type 5 displayed the previously documented pattern of gene transfer into both neurons and glial cells. Injection of Cy3 fluorescence-labelled CAV vector particles indicated that the vectors preferentially transduced neurons and suggested that the apparent neurotropism was not due to a restricted expression of the transgene in the context of the CAV vector. With a fluorescent conjugated CAV vector used in neuronal culture *in vitro,* and *in vivo,* the inventors demonstrate that this particular tropism resulted from a specific interaction of the LAV particles with neuritic and axonal processes.

### - Targeting of neuro-anatomical structures and accessibility of extended or deep structures:

For example, fig. 10, 11a, 11b, 12 show the pattern of gene transfer in neurons throughout respectively the dorsal hippocampus, the entorhinal cortex, (fig. 11 a, 11 b) and the substantia nigra pars compacta.

### - Retrograde axonal transport of viral particles:

The inventors also observed that the apparent high affinity of the CAV vectors for neuronal cells resulted in efficient gene transfer to neurons in the areas afferent to the injected structures. Injections in the hippocampus (fig. 10) permitted the transduction of afferent neurones of the entorhinal cortex (fig. 11 b). Injections in the striatum resulted in transduction of dopaminergic neurones in the substantia nigra compacta (fig. 12) and injections in the somatosensory cortex (fig. 13) lead to the transduction of neurones in the nucleus magnocellularis. All these data indicate a specific interaction of the CAV vector with a receptor located on neuronal processes. This interaction was thus demonstrated *in vitro* (fig. 9) and *in vivo.* The cellular entry of viral particles and their retrograde transport in neurons is demonstrated at 1h after intrahippocampic injection, leading to the presence of fluorescent conjugated particles closely associated to the nucleus of the neurons in entorhinal cortex (fig. 11a).

These data also indicated that CAV vectors are able to transfer genes to most neuronal cell types, without specific preferences. The high efficiency with which these vectors are able to transduce neurons may allow for the genetic modification of neuron populations whose cell body is remote from the injection site and difficult to access. Moreover, the inventors demonstrate here that the specific mechanism of entry in the neuronal cell for CAV vectors allows the genetic modification of neuronal cells, together with efficient gene transfer throughout entire neuro-anatomical structures.

### Example 4 : canine adenovirus-mediated gene transfer in human brain slices.

Human brain biopsies are recovered from the operating room as biological waste. Samples are immediately put in to artificial cerebral spinal fluid (ACSF) (52) and transported to the laboratory. The tissue was then cut using two scalpels into ~1 cm³ pieces before being sliced with a Sorval tissue chopper into ~200 pm section. The sections are then placed on nitro-cellulose filters (Millipore) with a pore size 45 pm. The filters were preincubated with 750 µl of ACSF and warmed to 37°C in 6-well dishes.

The slices are placed on the filters and 5 µl of solution containing 5 x 10⁸ particles of CAVGFP or AdGFP in ACSF are placed on the slice. The transduced slices are incubated at 37°WITH5% CO₂. The slices are fixed between day 3 and 5 and assayed for GFP expression by fluorescent microscopy. The results demonstrated that the CAV-2 vectors have a strong preference for neuronal types cells in healthy and tumour-derived tissue (figure 14). In addition the age and the region of the brain were the biopsy was taken of the tissue was independent of the tropism of the vectors. That is to say, regardless of the source or age of the tissue, CAV-2 vectors appeared to preferentially target neurons rather than the more abundant glial-derived cells in human brains.

### Example 5 : CAV mediated gene transfer in motoneurons after injection in muscle.

The inventors have compared the cell types transduced by CAVGFP and AdGFP.

### Experimental procedure:

7 new-born mice aged 4 days (strain = Swiss OF1) received in the left gastrocnemius a canine adenoviral vector and in the right gastrocnemius a human adenoviral vector. Both vectors contained the same expression cassette with the CMV promoter driving the expression of the GFP gene. An equal amount of viral particles was injected for both vectors. Two doses were assayed : 1.6 10¹⁰ vp (n = 4) and 3 10⁹ vp (n = 3).

The mice were sacrificed after weaning (24 days). Initially, an asymetry appeared in the size of muscles with an atrophy to the right compared to the side injected with the human adenovirus. Some of these mice exhibited limping (right side). Mice were anesthetized and perfused through the heart with a [PBS plus heparine] solution (20 ml) and a 2% paraformaldehyde solution (PAF). Gastrocnemia of both sides were removed. In the side injected with the human adenovirus, a spontaneous fluorescence of the muscles was observed, which was the clear sign of a high expression level. The sacral dorsolombar rachis flanked by the paravertebral muscles was removed, as well as the brain with the cerebellum and the higher part of the brain stem. All these elements were fixed overnight in 2% paraformaldehyde. The sacral dorsolombar cords were dissected the next day. Direct observation of muscles and cords at this stage with a fluorescence microscope showed: (1) high expression in the left muscles and weak expression to the right, which was later confirmed on frozen sections. (2) to the right, star-shaped cellular bodies that were GFP-positive (with sometimes prolongation of the signal up to the root, which strongly suggested a neuronal labelling).

After 72 hrs in 30 % sucrose, the organs were frozen. The blocks containing the cords were oriented by marking with a spot the cephalic extremity, and cut in section of 20 to 100 microns. The orientation of the sections was confirmed by comparing them to an atlas, as the spinal cord does not have the same aspect according to the level that is examined.

### Results:

All the sections were examined with a fluorescence microscope and the positive zone was located. In this zone, extending on several sections, the inventors noted a marking of large star-shaped cells with an extension far longer than the others, strongly evocating neuron, in the right anterior horn, but not in the left anterior horn (see figure 15).

### Conclusion:

The CAV vectors inefficiently transduce the muscular fibers, but preferentially transduce the motoneurons inervating the injected muscle with a 100-fold greater efficiency than the human adenovirus (which on the contrary efficiently infects the muscular fibers in new-borns)

### Example 6: CAV mediates gene transfer in the nasal cavity. Preferential transduction of sensory neurons inervating the olfactory bulb

5-week-old male Sprague Dawley rats were anaesthetised with ketamine a and xylasine and an aliquot of AdGFP or CAVGFP containing 5 x 10¹⁰ particles was placed in the nasal cavity with a Hamilton syringe. Rats were sacrificed 2 to 5 days later and the nasal epithelial and olfactory bulb fixed in 2% paraformaldehyde. The results demonstrate that AdGFP transduced the epithelial cells as well as the neurons innervating the olfactory bulb, whereas CAVGFP preferentially transduced only the neurons innervating the olfactory bulb (figure 16). The tropism in the nasal cavity mimicked that found when CAVGFP was injected in the muscle or in the central nervous system.

### Example 7 : Construction of variants canine adenovirus vectors

Improved canine adenovirus-derived vectors deprived of any overlap region in the E1A promoter with that integrated in the packaging cells DK/E1-28Z and DK28Cre :

The E1-transcomplementing cell lines described in examples 1 and 2 do not contain the CAV-2 inverted terminal repeat or the packaging signal found at the left end of the CAV-2 genome, but do contain a 55 bp of overlap in the E1A promoter with the vectors described in example 2. The inventors have generated other CAV vectors that do not contain an overlap in this region and all subsequent CAV vectors will not be able to generate RCA's via the *in vivo* mechanism characterised by Hehir et *al.* (17). Details on the contruction of such a vector are shown in figures 17 to 19.

The improved vector, CAV-GFP-2 (Figure 19), contains a single overlap of 713 bp in E2b with the cell line. Four high titre stocks (> 10¹² particles) of CAVGFP-2 were generated, using stock "a" as prestock for b, b for c etc. in order to amplify, if present, replication competent CAV-2 particle. 2 x 10⁹ DK cells, which are replication permissive for the wild type virus but not the vector, were infected with 2 x 10¹¹ vector particles. After 4 successive rounds of amplifications (4 -5 days each), the inventors were unable to detect replication competent CAV particles. These CAV-2 vector stocks and, more importantly, potential helper vectors for the production of gutless vectors, did not contain RCV.

### - Construction of a helper virus for the propagation of gutless CAV vectors :

In order to extend the size of the transgene inserted in the adenoviral vectors and to overcome some immunogenic problems with these vectors, some teams working on human adenoviral vectors have made vectors deleted of all viral coding sequences (referred to as "gutless" vectors). To propagate these gutless vectors, the viral functions must be provided in trans by another vector referred to as the "helper virus". The gutless vector is then separated from the helper virus by CsCI buoyant density (22). In order to reduce the contamination of the gutless vector by the helper virus, Parks et al. made helper vectors whose encapsidation signal was flanked by loxP sites. Co-infection of a Cre-expressing El-transcomplementation cell line by such a helper virus and a gutless vector will lead to preferential encapsidation of the gutless vector because of the excision of the encapsidation signal in the helper viral genome (22).

Using the same strategy, the inventors constructed helper vectors carrying loxP sites aroud both the encapsidation signal and the GFP transgene. Details of the construction of such a vector are shown in figures 20 to 23.

As Cre excision leads to an equilibrium between recombined and unrecombined genomes, further means to hinder the encapsidation of unrecombined genomes were tested. These include the generation of new helper viruses with a mutated encapsidation signal, according to the works of P. Hearing (44, 45) (figures 24 and 25).

The sequence of mutated encapsidation signals is shown in SEQ ID. 1 to 7.

The packaging capacity of some of these mutants was tested by competition experiments :

Adenoviruses that have a nonlethal mutation in the A repeats of the packaging signal (ψ) are able to be propagated and produced in quantity similar to viruses containing wild type ψ. Viruses that contain mutation in the packaging can be identified in a competition experiment when they are mixed with a virus containing the wild type ψ. The apparent limiting factors in packaging efficiency are the proteins that bind ψ.

This test encompasses infection of cells with a mutant and wild type virus/vector and identifying the percent of the mutant that is packaged versus the control. The inventors demonstrate here with two potential viruses that they have identified a CAV vector that contains a nonlethal mutation in the ψ. DK28Zeo cells were infected with 10 particles/cell of the test vectors (each of the test vectors contain an expression cassette encoding GFP) or 10 particles of the test vectors plus 100 particles/cell of a wild type ψ (CAV gal). These cells were collected 48 hours post-infection and the vectors recovered by 3 freeze/thaw cycles and the cellular debris removed by centrifugation. Twofold serial dilutions of this supernatant were incubated with DK28Zeo cells and analysed by flow cytometry 24 later.

As expected, the control infection containing CAVGFP showed a twofold reduction of the amplification of CAVGFP when mixed with CAVβgal. The packaging mutant CAVΔEhe showed an identical pattern compared to CAVGFP, demonstrating that this mutation did not affect packaging. Mutant CAVΔ337 had a greater than 10-fold inhibition of packaging when compared to amplified alone. These results (figure 26) demonstrate that the inventors have identified part of the CAV-2 packaging signal, CAVΔ337 contains a nonlethal packing mutation and CAVΔ337, which also contains a "floxed" packaging signal may be used to amplify the CAV gutless vectors in order to reduce the contamination with helper vectors.

### - construction of gutless canine vector genomes :

The construction of a first plasmid carrying a gutless CAV vector genome (pEJK25) is described in details in figures 27 to 37.

Cloning of small or large fragments into a plasmid that is greater than 25 kb (i.e. pEJK25) is exceptionally difficult because of the lack of compatible unique restriction enzyme sites. This in turn often forces the scientist to "blunt" the insert and the plasmid with DNA modifying enzymes (eg. Klenow or T4 DNA polymerase) and significantly reduce the chance of generating the plasmid.

In order to make the generation of gutless constructs more flexible, the inventors have devised a strategy to use homologous recombination in recBC sbcBC E. coli (6, 48 and 49) and a series of small pre-transfer plasmids (pEJK1, pEJK2, pEJK3 etc). This strategy allows the cloning of the desired transgene into a small plasmid with a large multiple cloning site.

The pre-transfer plasmid is chosen based on the size of the transgene because the size of the final gutless is preferentially within a limited size relative to the size of the helper vector (e.g., 23-25 kb). Each pre-transfer plasmid contains (a) the inverted terminal repeat and the packaging signal of CAV-2, (b) an expression cassette containing a promoter of choice (inducible, tissue specific, constitutive expression, etc.), an intron, a multiple cloning site, (c) a poly A signal, and (d) a fragment of the HPRT intron.

In each pre-transfer plasmid the HPRT intron sequence in this example is a ~1 KB fragment further along sequence. In other words, pEJK1 would have the first 1000 bp of the HPRT intron, pEJK2 would have bp 1001 to 2000, pEJK3 bp 2001-3000, etc. Once the transgene is cloned into the pre-transfer plasmid, this new construct is linearised (by choosing a site that cuts as close to the junction between the HPRT intron and the palsmid backbone). This choice of sites allows the largest region of overlap between the plasmid backbone (pPolyll) and the HPRT region cloned into the pre-transfer plasmid (pEJK25 is linearised with Mlu I). The greater the size of the transgene, the greater the deletion of the HPRT intron. As mentioned previously, the "stuffer" sequence may be any non-coding mammalian sequence.

The resulting "gutless plasmid and eventually gutless vector" then has a size that is (a) feasible to package in the CAV-2 capsid, and (b) is small enough to separate the gutless vector from the helper vector by CsCI buoyant density.
This strategy is summerized in figure 38.
This strategy was successfully illustrated by using pTCAV-7 and pEJK25 to insert the GFP transgene in the gutless construct, generating p25GFP (figure 39).

### - generation of gutless canine adenoviral vectors :

1 x 10⁶ DKCre cells were transfected with 4 µg of Asc I-digested p25GFP (the gutless construct) and 4 µg of Not I-digested pCAVBFP (helper vector expressing the BFP). The cells were rinsed the following day and incubated at 37°WITH5% C02 for 5 to 7 days. The cells were collected and the vectors released by 3 freeze / thaw cycles and 50% of the supernatant incubated overnight with 1 x 10⁶ DKCre cells. The media was removed, 10⁷ particles of CAVBFP was added and incubated overnight. This was repeated 4 to 5 times until no further increase in the percent GFP positive cells increased.

Amplification of the 25GFP was followed by fluorescent microscopy. Following the transfection ~10 to 15% of the cells were GFP positive. After the first amplification ~100 % GFP positive cells were detected. Following each of the first 4 amplifications a 2 to 4-fold increase in GFP positive cells was found. Following the 5^{th} amplification, no increase in the percentage of GFP positive cells was observed so a CsCI purified vector was produced. This was repeated 4 times until prep #10. Titration of this stock by flow cytometry demonstrated a concentration of 2.10⁹ particles/ml of 25GFP. 25GFPwas titred as described in example 2.

### Example 8 : approaches in some pathological states of the central nervous system:

### 1) Neurodegenerative diseases:

Transfer of a nucleotidic sequence with the aim to correct the genetic default when identified. (dominant or recessive neurodegenerative diseases).
- Huntington disease and other diseases related to the same genetic mechanism, i.e. the expansion of a repeated nucleotidic sequence.
   The interaction of the CAV vector with neuronal processes allows the adressing of a nucleotidic sequence to striatal neurons (mainly concerned by the degenerative process in Huntington disease) by the means of a stereotaxic injection into the projections sites of these neurons, namely the globus pallidus and the substantia nigra reticulata. Such a nucleotidic sequence can be designed with the aim of either correcting the genome itself or act at the level RNA processing and translation (anti-sens or rybozymes for example) or oppose the neurophysiological consequences of the degenerative process (to date essentially by transferring the gene of neurotrophic factors).
- Recessive diseases (familial forms of amyotrophic lateral sclerosis with mutated super oxyde dismutase or spinal muscular atrophy for example) resulting from the mutation or the absence of a gene, leading to a degeneration of motoneurons.
   The CAV vector allows the delivery of the deleted/mutated gene to some motoneurons either by direct injection in the anterior horn of the spinal cord or into the different neuronal tractus descending from or ascending to the brain, along which the vector particules are susceptible to diffuse and then reach the motoneurons along an extending proportion of the spinal cord. However, the great longer of the spinal axis may be an obvious limit for this approach. Another original approach can be designed with this vector in order to target the missing protein throughout all the anterior horn; indeed, the neuronal specificity of the CAV vector described here can lead to the efficient transduction of small nuclei located into the brain (for example the Red Nucleus) and innervating the whole motoneuronal population. The sequence of interest would be then the sequence of a fusion protein. The first protein will be a factor capable of translocation from one cell to another and the second protein would be the missing protein, thus addressed to adjacent cells, namely the motoneurons located at the ending of the Red Nuclei axons.
- Neurodegenerative diseases of unknown ethiology (exept the familial forms):
   The specific mechanism of retrograde axonal transport of the CAV vector described here will allow the targeting of defined and extended structures by the means of a stereotaxic injection into the projections sites described here. The efficient transduction of dopaminergic neurons in the substantia nigra (fig 12) is a concrete means of delivering a gene of interest to this anatomical zone which undergoes a devastating degeneration in Parkinson disease. This gene of interest can be to date the gene of neurotrophic factors, BDNF, CNTF, and more specially GDNF which seems to be a very potent factor for the survival of mesencephalic neurons. The high efficiency in transducing enthorinal cortex neurons (Fig 11) after a single injection of the CAV vector into the dentate gyrus inside the Hippocampus is of a great interest in the context of Alzheimer disease, together with the high efficiency of neuronal transduction in the hippocampus itself.

### 2) Disorders of the central nervous system with a degeneration process concerning primarily non neuronal cells:

Leucodystrophic disorders and multiple sclerosis: in such cases a great proportion of neurons transduced in key-zones of the brain with the CAV vector will enable the delivery to extended sites of either an anti-inflammatory molecule (anti-inflammatory cytokines) or an oligodendrocytes protecting factor (CNTF for example), of the missing protein.

### 3) Tumors of any origin located in the central nervous system.

In such cases neurons can be engineered by the CAV vector to secrete a fusion protein allowing the targeting of tumoral cells.

### 4) Metabolic disorders (mucopolysaccharidosis and other lysosomal storage diseases).

Whatever the mutated gene concerned, neurons can ben engineered by the CAV vector to secrete the specific missing enzyme, for example β glucuronidase (mucopolysaccharidosis type VII) or asparto-acylase (Canavan disease). The aim of such a strategy being also to correct the enzymatic defect throughout the brain and the spinal cord, the CAV vector can be then injected into different key sites, for example the hippocampus and the nucleus magnocellularis, as this latter nucleus is innervating the cortex in a widespread manner. A particular means to target the missing factor throughout the whole spinal axis will be to inject the CAV vector in some restricted nuclei located either in the mesencephale or the pons and sending their axons along the spinal cord.

### Example 9 : approaches in some pathological states of the peripheral nervous system:

### 1) Neuroblastoma:

These peripheral tumors are of neuronal origin. Specifically in this context, the neuronal specificity of the CAV vector will allow the delivery of a suicide gene mainly to the tumoral cells.

### 2) Peripheral neuropathies:

The specific mechanism of retrograde axonal transport of the CAV vector will allow to inject it in organs in order to engeneer the innervating motor and sensory neurons.

### 3) Pain treatment:

The same mechanism will allow to deliver to sensory neurons the gene of any factor capable to oppose the nociception process, for example, opiod receptors or endorphines.

### References

1. Altschul S.F., Madden T.L., Schaffer A.A., Zhang J., Zhang Z., Miller W., and Lipman D.J. 1997. Gapped BLAST and PSI-BLAST: a new generation of protein database search programs. Nucleic Acids Res. 25:3389-3402.
2. Ausubel F., Brent R., Kingston R., Moore D., Seidman J., Smith J., and Struhl K. (ed.). 1996. Current Protocols In Molecular Biology. John Wiley & Sons, Inc., New York.
3. Bergelson J.M., Cunningham J.A., Droguett G., Kurt E., Krithivas A., Hong J.S., Horwitz M.S., Crowell R.L., and Finberg R.W. 1997. Isolation of a common receptor for Coxsackie B viruses and adenoviruses 2 and 5. Science. 275:1320-1323.
4. Bett A.J., Prevec L., and Graham F.L. 1993. Packaging capacity and stability of human adenovirus type 5 vectors. J Virol. 67:5911-21.
5. Brown M., Rossier E., Carpenter B., and Anand C.M. 1991. Fatal adenovirus type 35 infection in newborns. Ped Inf Dis J. 10: 955-956.
6. Chartier C., Degryse E., Gantzer M., Dieterlé A., Pavirani A., and Mehtali M. 1996. Efficient generation of adenovirus vectors by homologous recombination in *Escherichia coli.* J Virol. 70:4805-4810.
7. Crystal R.G., Mastrangeli A., Sanders A., Cooke J., King T., Gilbert F., Henschke C., Pascal W., Herena J., and Harvey B.G. 1995. Evaluation of repeat administration of a replication deficient, recombinant adenovirus containing the normal cystic fibrosis transmembrane conductance regulator cDNA to the airways of individuals with cystic fibrosis. Hum Gene Ther. 6:667-703.
8. Crystal, R. G., N. G. McElvaney, M. A. Rosenfeld, C. Chu, A. Mastrangeli, J. G. Hay, S. L. Brody, H. A. Jaffe, N. T. Eissa, and C. Danel. 1994. Administration of an adenovirus containing the human CFTR cDNA to the respiratory tract of individuals with cystic fibrosis. Nature Genet. 8:42-51.
9. D'Ambrosio E., Del Grosso N., Chicca A., and Midulla M. 1982. Neutralizing antibodies against 33 human adenoviruses in normal children in Rome. J Hyg. 89:155-161.
10. Fallaux F., Kranenburg O., Cramer S., Howeling A., Van Ormondt H., Hoeben R., and Van der Eb A. 1996. Characterization of 911: a new helper cell line for the titration and propagation of early region 1-deleted adenoviral vectors. Hum Gene Ther. 7:215-222.
11. Fallaux, F. J., A. Bout, I. van der Velde, D. J. van den Wollenberg, K. M. Hehir, J. Keegan, C. Auger, S. J. Cramer, H. van Ormondt, A. J. van der Eb, D. Valerio, and R. C. Hoeben. 1998. New helper cells and matched early region 1deleted adenovirus vectors prevent generation of replication-competent adenoviruses. Hum Gene Ther. 9:1909-17.
12. Fasbender A., Lee J.H., Walters R.W., Moninger T.O., Zabner J., and Welsh M.J. 1998. Incorporation of adenovirus in calcium phosphate precipitates enhances gene transfer to airway epithelia In vitro and In vivo. J Clin Invest. 102:184-193.
13. Fejér, G., G. Berencsi, Z. Ruzsics, S. Belak, T. Linné, and I. Nasz. 1992. Multiple enlargements in the right inverted terminal repeat of the DNA of canine adenovirus type 2. Acta Microbiologica Hungarica. 39:159-168.
14. Flomenberg P., Piaskowski V., Truitt R.L., and Casper J.T. 1995. Characterization of human proliferative T cell response to adenovirus. J Inf Dis. 171:1090-1096.
15. Hamelin C., Jouvenne P., and Assaf R. 1986. Genotypic characterization of type-2 variants of canine adenovirus. Am J Vet Res. 47:625-630.
16. Hardy S., Kitamura M., Harris-Stansil T., Dai Y., and Phipps M.L. 1997. Construction of adenovirus vectors through Cre-lox recombination. J. Virol. 71:1842-1849.
17. Hehir K.M., Armentano D., Cardoza L.M., Choquette T.L., Berthelette P.B., White G.A., Couture L.A., Everton M.B., Keegan J., Martin J.M., Pratt D.A., Smith M.P., Smith A.E., and Wadsworth S.C. 1996. Molecular characterization of replication-competent variants of adenovirus vectors and genome modifications to prevent their occurrence. J Virol. 70:8459-8467.
18. Horwitz M.S. 1996. Adenoviruses., p. 2149-2171. *In* B. N. Fields and D. M. Knipe and P.M. Howley (ed.), Fields Virology, 3rd ed., vol. 2. Raven Press, New York.
19. Kaplan J.M., Pennington S., St George J.A., Woodworth L.A., Fasenbender A., Marshal J., Cheng S., Wadsworth S., Gregory R., and Smith A. 1998. Potential of gene transfer to the mouse lung by complexes of adenovirus vectors and polycations improves therapeutic potential. Hum Gene Ther. 9:1469-1479.
20. Klonjkowski B., Gilardi-Hebenstreit P., Hadchouel J., Randrianarison V., Boutin S., Perricaudet M., and Kremer E.J. 1997. A recombinant E1-deleted canine adenoviral vector capable of transduction and expression of a transgene in human-derived cells and in vivo. Hum Gene Ther. 8:2103-2115.
21. Knowles M.R., Hohneker K.W., Zhou Z., Olsen J.C., Noah T.L., Hu P.C., Leigh M.W., Engelhardt J.F., Edwards L.J., Jones K.R., et al. 1995. A controlled study of adenoviral-vector-mediated gene transfer in the nasal epithelium of patients with cystic fibrosis. N Engl J Med. 333.:823-831.
22. Kochanek S., Clemens P., Mitani K., Chen H., Chan S., and Caskey T. 1996. A new adenoviral vector: Replacement of all viral coding sequences with 28 kb of DNA independently expressing both full-length dystrophin and bgalactosidase. Proc Natl Acad Sci USA. 93:5731-5736.
23. Kremer E.J., and Perricaudet M. 1995. Adenovirus and adeno-associated virus mediated gene transfer. Br Med Bull. 51:31-46.
24. LeBaron C.W., Furutan N.P., Lew J.F., Allen J.R., Gouvea V., Moe C., and Monroe S.S. 1990. Viral agents of gastroenteritis. Public health importance and outbreak management. MMWR Morb Mortal Wkly Rep. 39:1-24.
25. Linne T. 1992. Differences in the E3 regions of the canine adenovirus type 1 and type 2. Virus Res. 23:119-133.
26. Lochmuller H., A. Jani, J. Huard, S. Prescott, M. Simoneau, B. Massie, G. Karpati, and G. Acsadi. 1994. Emergence of early region 1-containing replication-competent adenovirus in stocks of replication defective adenovirus recombinants during multiple passages in 293 cells. Hum Gene Ther. 5:1485-91.
27. Mastrangeli A, Harvey B.G., Yao J., Wolff G., Kovesdi I., Crystal R.G., and Falck-Pedersen E. 1996. "Sero-switch" adenovirus-mediated in vivo gene transfer: circumvention of anti-adenovirus humoral immune defenses against repeat adenovirus vector administration by changing the adenovirus serotype. Hum Gene Ther. 7:79-87.
28. Michou, A. I., H. Lehrmann, M. Saltik, and M. Cotten. 1999. Mutational analysis of the avian adenovirus CELO, which provides a basis for gene delivery vectors. J Virol. 73:1399-410.
29. Mittal S.K, Prevec L., Graham F., and Babiuk L.A. 1995. Development of a bovine adenovirus type 3-based expression vector. J Gen Virol. 76:93-102.
30. Mittereder N., March K.L., and Trapnell B.C. 1996. Evaluation of the concentration and bioactivity of adenovirus vectors for gene therapy. J Virol. 70:7498-7509.
31. Parks, R. J., and F. L. Graham. 1997. A helper-dependent system for adenovirus vector production helps define a lower limit for efficient DNA packaging. J Virol. 71:3293-8.
32. Piedra P.A., Poveda G.A., Ramsey B., McCoy K., and Hiatt P.W. 1998. Incidence and prevalence of neutralizing antibodies to the common adenoviruses in children with cystic fibrosis: implication for gene therapy with adenovirus vectors. Pediatrics. 101:1013-1019.
33. Rosenecker J., Harms K.H., Bertele R.M., Pohl-Koppe A., Mutius E., Adam D., and Nicolai T. 1996. Adenovirus infection in cystic fibrosis patients: implications for the use of adenoviral vectors for gene transfer. Infection. 24:5-8.
34. Ross D., and Ziff E. 1994. Defective processing of human adenovirus 2 late transcription unit mRNAs during abortive infections in monkey cells. Virol. 202:107-115.
35. Ross D., and Ziff E. 1992. Defective synthesis of early region 4 mRNAs during abortive adenovirus infections in monkey cells. J. Virol. 66:3110-3117.
36. Shenk T. 1996. Adenovirdea: The viruses and their replication, p. 2111-2148. *In* Fields BN and Knipe DM and Howley PM (ed.), Fields Virology. Raven Publishers, Philadelphia.
37. Sheppard., M., W. Werner, E. Tsatas, R. McCoy, S. Prowse, and M. Johnson. 1998. Fowl adenovirus recombinant expressing VP2 of infectious bursal disease virus induces protective immunity against bursal disease. Arch Virol. 143:915-30.
38. Stevenson S.C., Rollence M., Marshall-Neff J., and McClelland A. 1997. Selective targeting of human cells by a chimeric adenovirus vector containing a modified fiber protein. J Virol. 71:4782-4790.
39. Xu Z.Z, Hyatt A., Boyle D.B., and Both G.W. 1997. Construction of ovine adenovirus recombinants by gene insertion or deletion of related terminal region sequences. Virol. 230:62-71.
40. Yeh P., Dedieu J.F., Orsini C., Vigne E., Denefle P., and Perricaudet M. 1996. Construction and autonomous propagation of E1 1 & E4 deleted adenovector in a 293(E4+) cell line. J Virol. 70:559-565.
41. Zabner J., Couture L.A., Gregory R.J., Graham S.M., Smith A.E., and Walsh M.J. 1993. Adenovirus-mediated gene transfer transiently corrects the chloride transport defect in nasal epithelia of patients with cystic fibrosis. Cell. 75:207-216.
42. Zabner J., Petersen D.M., Puga A.P., Graham S.M., Couture L.A., Keyes L.D., Lukason M.J., St George J.A., Gregory R.J., Smith A.E., and Welsh M.J. 1994. Safety and efficacy of repetitive adenovirus-mediated transfer of CFTR cDNA to airway epithelia of primates and cotton rats. Nature Genet. 6:75-83.
43. Parks, R.J., Chen, L., Anton, M., Sankar, U., Rudnicki, M. A., and F. L. Graham, A helper-dependent adenovirus vector system: removal of helper virus by Cre-mediated excision of the viral packaging signal. Proc Natl Acad Sci U S A, 1996. 93(24): p. 13565-70.
44. Schmid, S.l. and P. Hearing, Bipartite structure and functional independence of adenovirus type 5 packaging elements. J Virol, 1997. 71(5): p. 3375-84.
45. Schmid, S.l. and P. Hearing, Cellular components interact with adenovirus type 5 minimal DNA packaging domains. J Virol, 1998. 72(8): p. 6339-47.
46. Choulika, A., V. Guyot, and J.F. Nicolas, Transfer of single genecontaining long terminal repeats into the genome of mammalian cells by a retroviral vector carrying the cre gene and the loxP site. J Virol., 1996. 70(3): p. 1792-8
47. Hanahan, D. Studies on transformation of Escherichia coli with plasmids. J. Mol. Biol., 1983, 166(4): p. 557-80.
48. Degryse, E. 1995. Evaluation of Escherichia coli recBC sbcBC mutants for cloning by recombination in vivo. J. Biotechnol. 39: 181-70
49. Degryse, E. 1996. In vivo intermolecular recombination in Escherichia coli: application to plasmid constructions. Gene. 170: 45-50.
50. Anton, M., and F. L. Graham. 1995. Site-specific recombination mediated by an adenovirus vector expressing the Cre recombinase protein: a molecular switch for control of gene expression. J. Virol. 69: 4600-6.
51. Morsy, M.A., M. Gu, S. Motzel, J. Zhao, J. Lin, Q. Su, H. Allen, L. Franlin, R.J. Parks, F.L. Graham, S. Kochanek, A.J. Bett, and C.T. Caskey. 1998. An adenoviral vector deleted for all viral coding sequences results in enhanced safety and extended expression of a leptin transgene. Proc Natl Acad Sci USA 95:7866-71.
52. Freese, A., M.G. Kaplitt, W. M. O' Connor, M. Abbey, D. Langer, P. Leone, M. J. O'Connor, and M.J. During. 1997. Direct gene transfer into human epileptogenic hippocampal tissue with an adeno-associated virus vector: implications for a gene therapy approach to epilepsy. Epilepsia. 38:759-66.

## Claims

1. Canine Adenovirus (CAV) vector obtainable by a process comprising the following steps :
a) co-transforming E. coli cells having recBC sbcBC phenotype by a first plasmid and a pre-transfer plasmid in conditions enabling their recombination by homologous recombination, in order to generate a transfer plasmid devoid from a functional E1 coding region, comprising the desired recombinant vector genome, wherein the first plasmid comprises the Inverted Terminal Regions (ITR) and the Packaging Signal (ψ) sequences of a CAV genome, and the pre-transfer plasmid includes the sequence whose insertion in the vector genome is desired, flanked by sequences homologous to sequences of the first plasmid surrounding the region of the first plasmid where the modification is desired,
b) isolating a DNA fragment essentially comprising the recombinant vector genome by enzyme restriction,
c) transfecting DK28Cre cells (CNCM 1-2293) that are rendered able to transcomplement this recombinant vector genome,
d) recovering and purifying the recombinant adenoviral particles produced.

2. Canine adenovirus (CAV) vector, which comprises:
a) a nucleotide sequence derived from Canine Adenovirus-2 strain Toronto A26/61 genomic sequence being devoid of the E1 region of the CAV genome, comprising the left and right inverted terminal repeat sequences (ITR) and the packaging signal (ψ) sequence,
b) an expression cassette comprising a nucleotide sequence to be transferred in target cells, said nucleotide sequence being under the control of regulation sequences including a promoter sequence.

3. Canine Adenovirus vector according to claim 1, wherein the CAV genomic sequences are derived from Canine Adenovirus-2 strain Toronto A26/61.

4. Canine Adenovirus vector according to claims 1 to 3 which comprises, the regulatory sequences and the sequences encoding strain Toronto A26/61 structure proteins of CAV-2 necessary for the synthesis of viral particles in E1-transcomplementing cells.

5. Canine Adenovirus vector according to anyone of claims 1 to 3, which comprises essentially all the nucleotide sequences encoding the viral functions of CAV-2 strain Toronto A26/61, except for the E1 coding region.

6. Canine Adenovirus vector according to anyone of claims 1 to 5, which comprises the the ITR and packaging signal (ψ) sequences fragment extending from nucleotide 1 to nucleotide 352 of the genomic sequence of the CAV-2 Toronto strain.

7. Canine Adenovirus vector according to anyone of claims 1 to 6, which comprises a second packaging signal (ψ) sequence.

8. Canine Adenovirus vector according to anyone of claims 1 to 7 wherein the ψ sequence is mutated.

9. Canine Adenovirus vector according to anyone of claims 1 to 8, wherein the expression cassette contains a nucleotide sequence to be transferred whose expression is driven by a viral promoter, a non viral or cellular promoter.

10. Canine Adenovirus vector according to anyone of claims 1 to 8, wherein the expression cassette is substituted for the E1 coding region of the CAV genome.

11. Canine Adenovirus vector according to anyone of claims 2 to 10, wherein the CAV-2 Toronto strain A26/61 genomic sequence is deleted from nucleotide 412 to nucleotide 2897.

12. Canine Adenovirus vector according to anyone of claims 1 to 3 and 5 to 11, which contains mammalian stuffer sequences.

13. Canine Adenovirus helper vector which is derived from a vector according to anyone of claims 1 to 12, and which further comprises lox sequences inserted in positions of the CAV genome which allow the deletion of the ψ sequence of the CAV genome, when the vector is contacted with a Cre recombinase.

14. Canine Adenovirus helper vector according to claim 13 which is devoid of an expression cassette.

15. Canine Adenovirus (CAV) vector according to anyone of claims 1 and 3 to 14, wherein said recombinant vector genome and transcomplementing cell line do not contain any overlapping sequence in the E1a region.

16. CAV vector genome such as that comprised in vector particles according to any of claims 1 to 15.

17. DNA construct comprising a CAV vector genome according to claim 16.

18. Plasmid comprising the genome of a canine adenovirus vector according to claim 1, which is pEJK25, p25GFP, pCAVGFP, or pCAVBFP.

19. Canine Adenovirus vector which is CAVGFP, deposited at the CNCM on Julyl9, 1999, under n° 1-2291.

20. Transcomplementing cell line for the production of Canine Adenovirus vector particles, which is a Dog Kidney (DK) cell line stably expressing the E1 region of the genomic sequence of a CAV-2 Manhattan strain, deposited at the CNCM on July 19, 1999, under n° I-2292.

21. Transcomplementing cell line according to claim 20 wherein the selection genes encoding for Neomycin and Zeocin resistance are substituted by other marker genes.

22. Transcomplementing cell line according to claim 20 or 21, which further expresses the Cre recombinase.

23. Transcomplementing cell line according to claim 22 which is the DK28Cre cell line deposited at the CNCM under n° I-2293 on July 19, 1999.

24. Transcomplementing cell line according to anyone of claims 20 to 23 transfected with the genome of a vector according to anyone of claims 1 to 18.

25. Use of a transcomplementing cell line according to anyone of claims 20 to 24 for the production of a CAV vector.

26. Use of Canine Adenovirus vector particles according to anyone of claims 1 to 18 or of their Canine Adenovirus vector genome for the preparation of a therapeutic composition for the treatment or modification of neuronal cells.

27. Use of Canine Adenovirus vector particles according to anyone of claims 1 to 18, or of their Canine Adenovirus vector genome, for the preparation of a therapeutic composition, for the targeted administration of a nucleotide sequence of therapeutic interest, in neuronal cells.

28. Use of Canine Adenovirus vector particles according to anyone of claims 1 to 18 or of their Canine Adenovirus vector genome for the preparation of a therapeutic composition capable of specifically interacting with neuritic terminations.

29. Use of Canine Adenovirus vector particles according to anyone of claims 1 to 18 or of their Canine Adenovirus vector genome for the preparation of a therapeutic composition for the transfer of a nucleotide sequence of interest *in vivo* in neuronal cells.

30. Use of a CAV vector according to anyone of claims 1 to 18 or of its CAV vector genome, for the preparation of a therapeutic composition for the treatment of a human patient presenting a humoral immunity against human adenovirus.

31. Use of Canine Adenovirus vector particles according to anyone of claims 1 to 18, or of their Canine Adenovirus genome for the screening of the delivery of a nucleotide sequence of interest in neuronal cells.

32. Canine adenoviral vector preparation that is contamined by less than 1 replication-competent particle in 2.10¹¹ viral particles.

33. Canine adenoviral vector preparation that contains more than 10¹³ viral particles/ml.

34. Process for the generation of recombinant Canine Adenoviral particles, comprising the following steps:
a) co-transforming E. coli cells by a first plasmid and a pre-transfer plasmid in conditions enabling their recombination by homologous recombination, in order to generate a transfer plasmid comprising the desired recombinant vector genome, wherein the first plasmid comprises the inverted terminal regions (ITR) and the packaging signal (ψ) sequences of a CAV genome, and the pre-transfer plasmid includes the sequence whose insertion in the vector genome is desired, flanked by sequences homologous to sequences of the first plasmid surrounding the region where the modification is desired,
b) isolating a DNA fragment essentially comprising the recombinant vector genome by enzyme restriction,
c) transfecting cells that are able to transcomplement this recombinant vector genome,
d) recovering the recombinant adenoviral particles produced.

35. Process according to claim 34, wherein said transcomplementation cells are a transcomplementing cell-line according to claims 20 to 23.

36. Process for the generation of gutless canine adenoviral vectors, comprising the following steps:
a) co-transforming E. coli cells by a first plasmid and a pre-transfer plasmid in conditions enabling their recombination by homologous recombination, in order to generate a transfer plasmid devoid of a functional E1 coding region, comprising the desired recombinant vector genome, wherein the first plasmid comprises the inverted terminal regions (ITR) and the packaging signal (ψ) sequences of a CAV genome, and the pre-transfer plasmid includes the sequence whose insertion in the vector genome is desired, flanked by sequences homologous to sequences of the first plasmid surrounding the region of the first plasmid where the modification is desired,
b) isolating a DNA fragment essentially comprising the recombinant vector genome by enzyme restriction,
c) transfecting cells that are rendered able to transcomplement this recombinant vector genome for the viral functions deleted in the first plasmid.
d) recovering and purifying the recombinant gutless adenoviral particles produced.

37. Process according to claim 36, wherein said recombinant canine adenoviral genome is deleted of essenitally all viral coding sequences and the transcomplementing cells are transcomplementing cells according to claims 20-23 transfected by a helper virus genome.

38. Process according to claim 35, wherein said recombinant canine adenoviral genome is deleted of essentially all viral coding sequences and the transcomplementing cells are E1-transcomplementing cells according to claims 20-23, infected by a helper virus devoid of E1.

39. Kit for the generation of recombinant CAV, comprising the following elements :
a) transcomplementation cells according to any of claims 20 to 23,
b) a first plasmid according to Claim 1, devoid of the E1 coding region of the CAV genome,
c) a pre-transfer plasmid, including sequences homologous to sequences of the first plasmid flanking the E1 deletion,
d) E. coli cells according to Claim 1.

40. Kit for the generation of gutless CAV vectors, comprising the following elements :
a) transcomplementation cells according to any of claims 22 or 23,
b) a first plasmid according to Claim 1, devoid of all the viral coding sequences of the CAV genome,
c) a pre-transfer plasmid, including sequences homologous to sequences of the first plasmid,
d) E. coli cells according to Claim 1.
